# EUROPEAN PATENT APPLICATION

(11) **EP 1 176 141 A1**
(43) Date of publication of application: **30.01.2002**
(21) Application number: 00906592.1
(22) Date of filing: 01.03.2000
(51) Int. Cl.: C07D 213/73, C07D 417/06, C07D 401/06, C07D 413/06, C07D 233/88, A61K 31/44, A61K 31/4439, A61K 31/444, A61K 31/506, A61K 31/501, A61K 31/5355, A61K 31/427, A61K 31/4168

(54) **HETEROCYCLIC COMPOUNDS HAVING EFFECT OF ACTIVATING NICOTINIC ACETYLCHOLINE $g(a)4$g(b)2 RECEPTOR**

(30) Priority: 05.03.1999 JP 5799399
(71) Applicant: SUNTORY LIMITED, Osaka-shi, Osaka 530-8203 (JP)
(72) Inventor: IMOTO, Masahiro, Nishinomiya-shi, Hyogo 662-0054 (JP); IWANAMI, Tatsuya, Ashikaga-shi, Tochigi 326-0824 (JP); AKABANE, Minako, Ibaraki-shi, Osaka 567-0801 (JP); TANI, Yoshihiro, Ibaraki-shi, Osaka 567-0843 (JP)
(74) Representative: VOSSIUS & PARTNER
(86) International application number: JP0001190
(87) International publication number: WO0053582

(57) **Abstract**

There is provided heterocyclic compounds of the following formula (I): 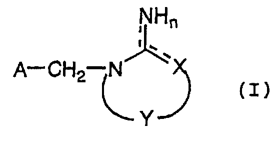 in which,
A is optionally substituted aryl group or optionally substituted heterocyclic group;
X is oxygen atom, sulfur atom, carbon atom or nitrogen atom;
   dotted line shows either presence or absence of bond;
n is integer of 1 or 2; and
Y represents alkylene bond and so on;
or a pharmaceutically acceptable salt thereof.

These compounds have good affinity to α4β2 nicotinic acetylcholine receptors and activate the same to thereby exert a preventive or therapeutic effect on cerebral dysfunction.

## Description

### TECHNICAL FIELD

The present invention relates to compounds showing affinity to nicotinic acetylcholine receptors and activating the same. The compounds of the present invention are useful for preventing or treating of neurodegenerative diseases such as Alzheimer's disease and Parkinson's disease, dementia such as cerebrovascular dementia, motor ataxia such as Tourette's syndrome, neurosis during chronic cerebral infarction stage, neuropathy and mental disorder such as anxiety and schizophrenia and cerebral dysfunction caused by cerebral injury.

### BACKGROUND ART

It has been widely known that nicotine exerts a wide variety of pharmacological effects. These include, for example, cholinergic nervous activation as the effect on central nervous system such as facilitation of acetylcholine release [De Sarno P. & Giacobini E., *J. Neurosci*. *Res.,* 22, 194-200 (1984)], and further, activating effect on monoaminergic nervous system [Levin E. D. & Simon B. B., *Psychopharmacology*, 138, 217-230 (1998)].

It has been also reported that nicotine possesses lots of very useful cerebral function improving effects such as increasing cerebral blood flow and glucose uptake rate in brain [Decker M. W. et al., *Life Sci*., 56, 545-570 (1995)].

It has been further reported that nicotine inhibits amyloid formation of β-peptides which is believed to be the cause of neuronal cell death during Alzheimer's disease [Salomon A. R. et al., *Biochemistry,* 35, 13568-13578 (1996)], and have cell protective effects on neuronal cell death induced by β-amyloid (Aβ) [Kihara T. et al., *Ann. Neurol.*, 42, 156-163 (1997)]. Recent studies suggest the possibility of nicotine being a remedy for the inflammatory colitis [Sandborn W. J. et al., *Ann. Intern. Med.*, 126, 364 (1997)].

On the other hand, it is acknowledged that in the patients of Alzheimer's disease, the degeneration of acetylcholinergic neurons known to be one of the important nervous systems responsible for cognition such as attention, learning, memory and recognition, is altered and thus nicotinic acetylcholine receptors in the cerebral cortex and hippocampus are drastically decreased [Nordberg A. et al., *J. Neurosci*. *Res.,* 31, 103-111 (1992)].

It is reported that there is a possibility of treating Alzheimer's disease by activating nicotinic acetylcholine receptors to recover the function of acetylcholine nervous system by agonists or modulators of nicotinic acetylcholine receptors [Newhouse P. A. et al., *Psychopharmacology*, 95, 171-175 (1988)].

Nicotinic acetylcholine receptors belong to ion channel neurotransmitter receptors composed of five subunits. That is, agonists such as acetylcholine, nicotine and the like are bound to receptors to activate and open the channels thereof, thus causing the influx of cationic ion such as sodium ion from extracellular to result the cell excitation [Galzi J. L. & Changeux J. P., *Neuropharmacology*, 34, 563-582 (1995)]. Aforementioned agonists such as acetylcholine, nicotine and the like show its effect by binding to the specific site existing in α subunit so-called agonist binding site.

It is known, on the other hand, that compounds such as galantamine and so on which activate cells by potentiating the effects of acetylcholine, have no agonist effect at nicotinic acetylcholine receptors directly. These compounds show their effects through allosteric site which is clearly different from the agonist binding sites [Schrattenholz A. et al., *Mol*. *Pharmacol*., 49, 1-6 (1996)].

Mentioned above, compounds capable to activate nicotinic acetylcholine receptors indirectly are called modulators and it is expected to be the practical medicine for treatment of the various neurological diseases [Lin N. -H & Meyer M. D., *Exp*. *Opin*. *Ther*. *Patents, 8,* 991-1015 (1998)].

The terms "agonists" and "modulators" are used in these definitions in the present specification.

Nicotinic acetylcholine receptors are believed to participate not only in Alzheimer's disease, but also in neurodegenerative diseases such as Parkinson's disease, and many of the neurosis and psychosis such as dementia, anxiety, schizophrenia and so on [Barrantes F. J., in *The Nicotic Acetylcholine Receptor,* ed. Barrantes F. J., Springer, 1997, p175-212; Lena C. & Changeux J. *-P., J. Physiol*. *(Paris)*, 92, 63-74 (1998)].

Especially, since it is known that cerebral blood flow of the patients suffering from cerebrovascular dementia caused by cerebral infarction is decreased [Takagi Shigeharu, *Gandai Iryo*, 28, 1157-1160 (1996); Tachibana H. et al., *J*. *Gerontol*., 39, 415-423 (1984)], there seems to be the possibility of agonists of nicotinic acetylcholine receptors or the modulators possessing cerebral blood flow increasing effect to be applied to medicine in this area of treatment. Furthermore, recent study revealed that agonists of nicotinic acetylcholine receptors and modulators thereof show analgesic activities [Bannon A. W. et al., *Science*, 279, 77-81 (1998)].

Nicotine itself surely affects as agonist of nicotinic acetylcholine receptors. For example, after administration of nicotine to patients of Alzheimer's disease, recoveries of their attention or the short-term memory were observed, and also the symptoms of their disease were improved [Newhouse P. A. et al., *Drugs & Aging*, 11, 206-228 (1997)]. Nevertheless, nicotine also possesses disadvantages such as widely recognized addiction, as well as low bioavailability and severe side effects to the cardiovascular system.

Therefore, there have been great expectation to develop nicotinic acetylcholine receptors agonists or modulators as medicine in place of nicotine which has no addiction, high bioavailability, and less side effects on cardiovascular system [Maelicke A. & Albuquerque E. X., *Drug Discovery Today*, 1, 53-59 (1996); Holladay M. W. et al., *J*. *Med*. *Chem*., 40, 4169-4194 (1997)].

There are some subtypes known as nicotinic acetylcholine receptors [Shacka J. J. & Robinson S. E. T., *Med*. *Chem*. *Res.,* 1996, 444-464], and mainly α4β2 subtype receptors exist in central nervous system. Furthermore, there exist α1β1γδ (or α1β1εδ) subtype receptors in the neuromuscular junction of motor neurons, and α3β4 subtype receptors in ganglion of autonomic nervous system and adrenal.

Activation of cholinergic nervous system and increasing effect of cerebral blood flow are believed to occur though α4β2 subtype receptors in central nervous system, and above mentioned effects of nicotine on cardiovascular system are induced by affecting receptor subtypes exist in peripheral nervous system.

Therefore, it may be extremely useful to develop compounds which have no affinity at α1β1γ*δ* subtype nor α3β4 subtype receptors but selectively affects α4β2 subtype receptors, as medicine having no side effects.

In these circumstances, there have been many proposals to develop selective agonists or modulators at nicotinic acetylcholine receptors of central nervous system as practical medicine. These include, for example, the compound such as ABT-418 [Arneric S. P. et al., *J. Pharmacol*. *Exp*. *Ther*., 270, 310-318 (1994); Decker M. W. et al., *J. Pharmacol*. *Exp*. *Ther*., 270, 319-328 (1994)], ABT-089 [Sullivan J. P. et al., *J*. *Pharmacol*. *Exp*. *Ther*., 283, 235-246 (1997); Decker M. W. et al., *J. Pharmacol*. *Exp*. *Ther*., 283, 247-258 (1997)], GTS-21 [Arendash G. W. et al., *Brain Res*., 674, 252-259 (1995); Briggs C. A. et al., *Pharmacol*. *Biochem*. *Behav*., 57, 231-241 (1997)], RJR-2403 [Bencherif M. et al., *J. Pharmacol*. *Exp*. *Ther*., 279, 1413-1421 (1996); Lippiello P. M. et al., *J. Pharmacol*. *Exp*. *Ther*., 279, 1422-1429 (1996)], SIB-1508Y [Cosford N. D. P. et al., *J*. *Med*. *Chem*., 39, 3235-3237 (1996); Lloyd G. K. et al., *Life Sci*., 62, 1601-1606 (1995)], SIB-1553A [Lloyd G. K. et al., *Life Sci*., 62, 1601-1606 (1995)] and so on.

In European Patent Publication EP679397-A2, substituted amine derivatives represented by the following formula were proposed for the medicine for prevention and treatment of cerebral dysfunction. 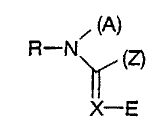 in which,
R represents hydrogen, optionally substituted acyl, alkyl, aryl, aralkyl, heteroaryl or heteroarylalkyl radicals;
A represents a monofunctional group of the hydrogen, acyl, alkyl or aryl series or represents a bifunctional group which is linked to the radical Z;
E represents an electron-withdrawing radical;
X represents -CH= or =N- radicals, it being possible for the -CH= radical to be linked to Z radical instead of H atom;
Z represents a monofunctional group of alkyl, -O-R, -S-R or -NR₂ series or represents a bifunctional group which is linked to A radical or X radical.

However, there is no description in the above-mentioned patent publication that these compounds can selectively activate α4β2 nicotinic acetylcholine receptors.

On the other hand, "imidacloprid", as a pesticide, is known to have similar skeleton as the compounds of the present invention. It is confirmed that imidacloprid electrophysiologically affects as partial agonist at nicotinic acetylcholine receptors of PC12 cell [Nagata K. et al., *J. Pharmacol*. *Exp*. *Ther*., 285, 731-738 (1998)], and imidacloprid itself or its metabolites and their analogues possess affinity to nicotinic acetylcholine receptors in mouse brain [Lee Chao S. & Casida E., *Pestic*. *Biochem*. *Physiol*., 58, 77-88 (1997); Tomizawa T. & Casida J. E., *J. Pharmacol*., 127, 115-122 (1999); Latli B. et al., *J. Med*. *Chem*., 42, 2227-2234 (1999)], however, there is no report of imidacloprid derivatives selectively activating α4β2 nicotinic acetylcholine receptors.

Japanese Laid-open Patent Publication Number Hei 10-226684 disclosed [N-(pyridinylmethyl)heterocyclic]ylideneamine compounds represented by the following formula, pharmaceutically acceptable salts and prodrugs thereof. 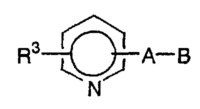 in which,
A represents -CH(R)-;
R³ represents hydrogen atom or optionally substituted C₁-C₆ alkyl; and
B represents the group of the following formula:

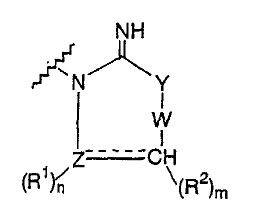

Nevertheless, among the compounds disclosed in said patent publication possess weak affinity to nicotinic receptors; however, there is no disclosure that these compounds have selective activating effect at α4β2 nicotinic acetylcholine receptors of central nervous systems and act as agonists or modulators of nicotinic acetylcholine receptors.

As mentioned above, there had been many attempts to develop agonists or modulators selectively activating α4β2 nicotinic acetylcholine receptors of central nervous system via oral administration, but none were satisfactory.

Therefore, the present invention provides therapeutic or preventing agents for treatment of diseases which may be prevented or cured by activating nicotinic acetylcholine receptors, having capabilities of binding selectively with α4β2 nicotinic acetylcholine receptors of central nervous system, and having no undesirable side effects in cardiovascular system such as hypertension or tachycardia.

More specifically, the present invention provides medicaments for preventing or treating various diseases, which may be prevented or cured by activating nicotinic acetylcholine receptors, such as dementia, senile dementia, presenile dementia, Alzheimer's disease, Parkinson's disease, cerebrovascular dementia, AIDS-related dementia, dementia in Down's syndrome, Tourette's syndrome, neurosis during chronic cerebral infarction stage, cerebral dysfunction caused by cerebral injury, anxiety, schizophrenia, depression, Huntington's disease, pain and so on.

### DISCLOSURE OF THE INVENTION

Through extensive investigations of researching compounds having capabilities of binding selectively with α4β2 nicotinic acetylcholine receptors of central nervous system, the present inventors discovered that the compounds represented by the formula (I) mentioned below and pharmaceutically acceptable salts thereof possess high affinity to nicotinic acetylcholine receptors in central nervous system, and activate said receptors as agonists or modulators.

Accordingly, as one aspect of the present invention, it is provided the heterocyclic compounds represented by the following formula (I): 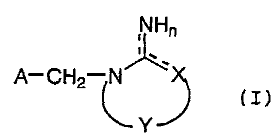 wherein:
A is optionally substituted aryl group; or optionally substituted heterocyclic group;
X is oxygen atom; sulfur atom; carbon atom; or nitrogen atom;
   dotted line shows either presence or absence of bond;
n is integer of 1 or 2; and
Y is,
   (1) in the case of X is oxygen atom, group -Y-X- is -CH₂-CH₂-O- or -CH₂-CH₂-CH₂-O-;
   (2) in the case of X is sulfur atom, group -Y-X- is-CH(R¹)-CH₂-S-, -C(R²)=C(R³)-S- or -CH₂-CH₂-CH₂-S- (in which, R¹, R² and R³ are hydrogen atom; C₁-C₄ alkyl group; or optionally substituted phenyl group);
   (3) in the case of X is carbon atom, group -Y-X- is -CH₂-CH₂-CH₂-, -CH=C(R⁴)-C(R⁵)=C(R⁶)-, -CH₂-CH₂-CH₂-CH₂-, or -N=C(R⁷)-CH=CH- (in which, R⁴, R⁵, R⁶ and R⁷ are hydrogen atom; C₁-C₄ alkyl group; optionally substituted phenyl group; halogen atom; or nitro group); and,
   (4) in the case of X is nitrogen atom, group -Y-X- is-CH₂-CH₂-NH-, -CH₂-CH₂-CH₂-NH-, -CH=C(R⁸)-N= or -CH-C(R⁹)-CH=N- (in which, R⁸ and R⁹ are hydrogen atom; or optionally substituted phenyl group);
      or pharmaceutically acceptable salts thereof.

Still another aspect of the present invention, it is provided activating agents for α4β2 nicotinic acetylcholine receptors containing the heterocyclic compounds of the formula (I) or pharmaceutically acceptable salt thereof as active ingredients.

As still further aspect of the present invention, it is provided that use of the heterocyclic compounds of the formula (I) or pharmaceutically acceptable salt thereof for treating or preventing of cerebral circulation disease, neurodegenerative disease and the like.

### BEST MODE FOR CARRYING OUT THE INVENTION

Examples of pharmaceutically acceptable salt include inorganic acid salt such as hydrochloric acid salt, hydrobromic acid salt, sulfuric acid salt, phosphoric acid salt and the like, and organic acid salt such as fumaric acid salt, maleic acid salt, oxalic acid salt, citric acid salt, tartaric acid salt, malic acid salt, lactic acid salt, succinic acid salt, benzoic acid salt, methanesulfonic acid salt, p-toluenesulfonic acid salt and the like.

The group represented by "A" in the compound of the formula (I) is optionally substituted aryl group or optionally substituted heterocyclic group, and preferable examples of said optionally substituted aryl group include phenyl; naphthyl and the like. Examples of suitable substituent of substituted aryl group include C₁-C₄ lower alkyl, halogen atom, nitro group, cyano group and the like, and therefore, examples of said substituted aryl group include methylphenyl, trifluoromethylphenyl, chlorophenyl, dichlorophenyl, nitrophenyl, cyanophenyl and the like.

The term "heterocyclic group" represented by "A" may be 5 or 6 membered heterocyclic group or condensed heterocyclic group thereof containing the same or different 1 to 3 hetero atom(s) such as sulfur, nitrogen, oxygen atom(s), and examples include thiophene, furan, pyran, pyrrole, pyrazole, pyridine, pyrimidine, pyrazine, pyridazine, imidazole, oxazole, isoxazole, thiazole, isothiazole, quinoline, isoquinoline, azaindole, tetrahydropyrimidine and the like.

Examples of suitable substituent of substituted heterocyclic group include C₁-C₄ lower alkyl, halogen atom and the like, and therefore, examples of said substituted heterocyclic group include 2-methylpyridine, 2-chloropyridine, 2-fluoro-pyridine, 2-bromopyridine, 3-bromopyridine, 2,3-dichloropyridine, 2-chlorothiazole, 3-methylisoxazole and the like.

The dotted line in the compound of the formula (I) shows either presence or absence of bond, and has following meanings in relation to number "n"; that is, in the case number "n" is 1, double bond is located between carbon atom of heterocyclic ring and exocyclic nitrogen atom, and so said nitrogen atom corresponds to imino group, and in another case number "n" is 2, double bond is located between carbon atom of heterocyclic ring and "X" which refers carbon or nitrogen atom, and then exocyclic nitrogen atom corresponds to amino group as substituent of heterocyclic ring.

The group represented by "X" in the compound of the formula (I) stands for oxygen atom, sulfur atom, carbon atom or nitrogen atom, and the "X" is combined with "Y" to constitute the partial component represented by "-Y-X-", which has follow meanings.
(1) in the case of "X" is oxygen atom, the term "-Y-X-" is -CH₂-CH₂-O- or -CH₂-CH₂-CH₂-O-:
(2) in the case of "X" is sulfur atom, the term "-Y-X-" is -CH(R¹)-CH₂-S-, -C(R²)=C(R³)-S- or -CH₂-CH₂-CH₂-S-,
   (in which, R¹, R² and R³ are hydrogen atom; C₁-C₄ alkyl group; or optionally substituted phenyl group);
(3) in the case of "X" is carbon atom, the term "-Y-X-" is -CH₂-CH₂-CH₂-, -CH=C(R⁴)-C(R⁵)=C(R⁶)-, -CH₂-CH₂-CH₂-CH₂- or -N=C(R⁷)-CH=CH-
   (in which, R⁴, R⁵, R⁶ and R⁷ are hydrogen atom; C₁-C₄ alkyl group; optionally substituted phenyl group; halogen atom; or nitro group);
(4) in the case of "X" is nitrogen atom, the term "-Y-X-" is -CH₂-CH₂-NH-, -CH₂-CH₂-CH₂-NH-, -CH=C(R⁸)-N= or -CH=C(R⁹)-CH=N -
   (in which, R⁸ and R⁹ are hydrogen atom; or optionally substituted phenyl group), and the like.

The term "C₁-C₄ alkyl group" represented by R¹, R², R³, R⁴, R⁵, R⁶, R⁷, R⁸, and R⁹ include methyl, ethyl, propyl, isopropyl, butyl, isobutyl, sec-butyl, tert-butyl and the like. The term "optionally substituted phenyl group" includes non-substituted phenyl group, C₁-C₄ lower alkyl such as methyl, ethyl and the like, or phenyl group which is substituted by halogen atom. The term "halogen atom" includes fluorine, chlorine, bromine and iodine.

The heterocyclic compounds represented by the formula (I) of the present invention can be prepared in accordance with the various synthetic processes such as following Process 1 to 4.

In the following reaction schemes, the groups A, X, Y and n have the same meanings mentioned above.

### Process 1:

In accordance with the following reaction scheme, the compound of the formula (II) is reacted with the compound of the formula (III) to obtain the compound (I) of the present invention. 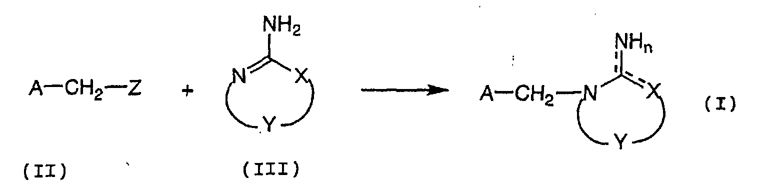 wherein, "Z" is leaving group which accelerates the reaction with nitrogen atoms of heterocyclic ring, such as halogen atom, p-toluenesulfonyloxy, methanesulfonyloxy, trifluoromethane-sulfonyloxy, acyloxy, substituted acyloxy groups and so on.

The compound (III) to be used in this reaction can be commercially available or can be easily prepared from known compounds by using common methods.

The reaction of the compound (II) with the compound (III) to obtain the compound (I) can be usually carried out in an appropriate solvent such as alcohol solvent, ketone solvent, nitrile solvent, ester solvent, amide solvent, hydrocarbon solvent and ether solvent or the mixture thereof in the presence of organic base or inorganic base if necessary, under the temperature ranging from -20°C to the refluxing temperature of the solvent to be used.

Examples of alcohol solvent include methanol, ethanol, propanol, 2-propanol, 2-methyl-2-propanol and the like. Examples of ketone solvent include acetone, methyl ethyl ketone and the like. Examples of nitrile solvent include acetonitrile, propionitrile and so on, and ester solvent includes ethyl acetate. Examples of amide solvent include N,N-dimethylformamide, N,N-dimethylacetamide, N-methylpyrrolidone, hexamethylphosphoramide and the like. Examples of hydrocarbon solvent include aromatic hydrocarbon such as benzene, toluene and the like, or aliphatic hydrocarbon such as pentane, hexane and the like. Examples of ether solvent include diethyl ether, dimethoxyethane, tetrahydrofuran, 1,4-dioxane and the like.

Examples of organic base to be used in the reaction may include triethylamine, collidine, lutidine, potassium tert-butoxide and the like, and inorganic base to be used in the reaction include potassium carbonate, sodium carbonate, sodium hydrogencarbonate, sodium hydroxide, potassium hydroxide and the like.

### Process 2:

The compound (I) can be obtained by removing the nitro group of the compound (IV) in accordance with the following reaction scheme.

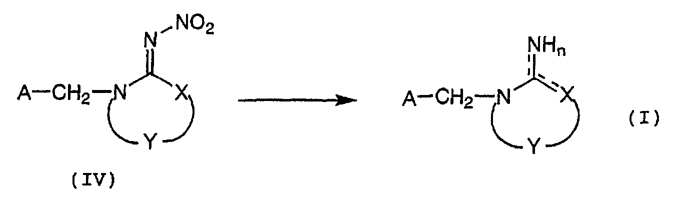

The compound (IV) to be used in this reaction can be prepared in accordance with the known method (Moriya K. et al., *J. Pesticides Sci*., 18, 119-123 (1993)). Removing the nitro group of the compound (IV) can be conducted by using common method such as deprotection of peptides including nitroarginine.

This removing reaction of the nitro group of the compound (IV) can generally be carried out by treating with a reducing reagent in water, or in alcohol solvent, amide solvent, acid solvent alone, or in the mixture solvent thereof, at the temperature ranging from -20°C to 50°C, in the presence of organic or inorganic salt having buffer action, if necessary.

Examples of alcohol solvent include methanol, ethanol, propanol, 2-propanol, 2-methyl-2-propanol and the like. Examples of amide solvent include N,N-dimethylformamide, N,N-dimethylacetamide, N-methylpyrrolidone, hexamethylphosphoramide and the like. Examples of acid solvent include formic acid, acetic acid, propionic acid, trifluoroacetic acid, hydrochloric acid and the like. Examples of organic or inorganic salt having buffer action include ammonium acetate, triethylamine, pyridine, phosphate salts and the like. Preferable reducing reagent is titanium (III) chloride.

### Process 3:

The compound (I) can be obtained by reacting the compound (V) with the compound (VI) to derive the intermediate (VII) and cyclizing the resultant compound (VII) in accordance with the following reaction scheme. 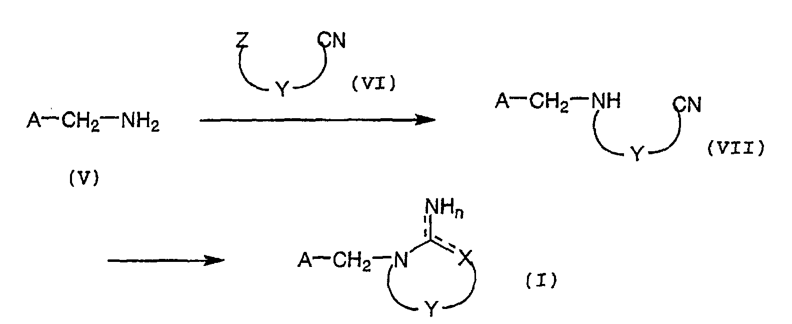 wherein, Z has the same definition as mentioned above.

The compound (V) to be used in this reaction can be commercially available or prepared in accordance with the known method to the person skilled in the art. Examples of the compound (VI) include 4-bromobutyronitrile or 5-bromovaleronitrile.

This reaction to obtain intermediate (VII) by reacting the compound (V) and the compound (VI) can generally be carried out in an appropriate solvent such as alcohol solvent, ketone solvent, nitrile solvent, ester solvent, amide solvent, hydrocarbon solvent and ether solvent or the mixture thereof in the presence of organic base or inorganic base if necessary, under the temperature ranging from -20°C to the refluxing temperature of the solvent to be used.

Examples of alcohol solvent include methanol, ethanol, propanol, 2-propanol, 2-methyl-2-propanol and the like. Examples of ketone solvent include acetone, methyl ethyl ketone and the like. Examples of nitrile solvent include acetonitrile, propionitrile and the like. Examples of ester solvent include ethyl acetate. Examples of amide solvent include N,N-dimethylformamide, N,N-dimethylacetamide, N-methylpyrrolidone, hexamethylphosphoramide and the like. Examples of hydrocarbon solvent include aromatic hydrocarbon such as benzene and toluene and the like, or aliphatic hydrocarbon such as pentane and hexane and the like. Examples of ether solvent include diethyl ether, dimethoxyethane, tetrahydrofuran, 1,4-dioxane and the like.

Examples of organic base to be used in the reaction include triethylamine, collidine, lutidine, potassium tert-butoxide and the like, and inorganic base to be used in the reaction include potassium carbonate, sodium carbonate, sodium hydrogencarbonate, sodium hydroxide, potassium hydroxide and the like.

Conversion of the compound (VII) into the compound (I) by cyclization can generally be carried out in hydrocarbon alone as reaction solvent, or in the mixture solvent thereof, at the temperature ranging from room temperature to 200°C, in the presence of aluminum reagent, if necessary. This reaction can also be carried out without any solvent.

Examples of hydrocarbon used as solvent include aromatic hydrocarbon such as benzene, toluene and the like, or aliphatic hydrocarbon such as pentane, hexane and the like.

Examples of aluminum reagent can be listed as trimethylaluminum, triethylaluminum, dimethylaluminum chloride, diethylaluminum chloride, ethylaluminum dichloride and the like.

### Process 4:

The compound (I) can be obtained by the reaction between the compound (VIII) and the compound (IX) in accordance with the following reaction scheme. 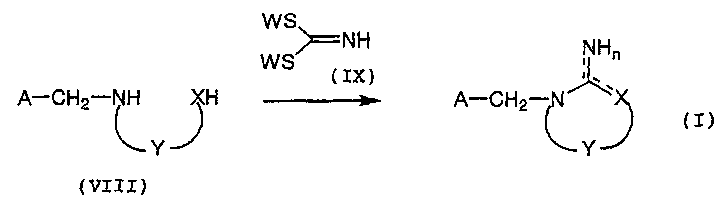 wherein, W represents alkyl group, substituted alkyl group, aryl group or substituted aryl group.

The compound (VIII) to be used in this reaction can be prepared in accordance with the known method (Moriya K. et al., *J*. *Pesticides Sci*., 18, 119-123 (1993)). The compound (IX) to be used in this reaction can be prepared in accordance with the known method (Habicher W-D. & Mayer R., *Z*. *Chem.,* 12, 459-460 (1968)).

This reaction to obtain the compound (I) from the compound (VIII) and the compound (IX) can generally be carried out in alcohol solvent, amide solvent, hydrocarbon solvent, ether solvent alone, or in the mixture solvent thereof, at the temperature ranging from room temperature to the refluxing temperature of the solvent to be used, in the presence of organic or inorganic salt, if necessary.

Examples of alcohol solvent include methanol, ethanol, propanol, 2-propanol, 2-methyl-2-propanol and the like. Examples of amide solvent include N,N-dimethylformamide, N,N-dimethyl-acetamide, N-methylpyrrolidone, hexamethylphosphoramide and the like. Examples of hydrocarbon solvent include aromatic hydrocarbon such as benzene, toluene and the like, or aliphatic hydrocarbon such as pentane, hexane and the like. Examples of ether solvent include dimethoxyethane, tetrahydrofuran, 1,4-dioxane and the like.

Examples of organic base to be used in the reaction include triethylamine, collidine, lutidine, potassium tert-butoxide and the like, and inorganic base to be used in the reaction include potassium carbonate, sodium carbonate, sodium hydrogencarbonate, sodium hydroxide, potassium hydroxide and the like.

The compound of the formula (I) of the present invention thus obtained can be converted to pharmaceutically acceptable salt with various kinds of organic or inorganic acids mentioned above, if necessary. Furthermore, the compound (I) of the present invention can also be purified by the conventional manner, such as recrystallization, column chromatography and the like.

When the compounds of the formula (I) of the present invention exist in isomer forms, each isomer *per se* is separated from each other by the conventional manner. Therefore, it is understood that each isomers *per se*, as well as isomeric mixture, shall be included in the compounds of the present invention.

The compounds of the formula (I) of the present invention bind selectively to nicotinic acetylcholine receptors in central nervous system, and activate said receptors as agonists or modulators. Therefore, these compounds are useful as medicaments for preventing or treating various diseases, such as dementia, senile dementia, presenile dementia, Alzheimer's disease, Parkinson's disease, cerebrovascular dementia, AIDS-related dementia, dementia in Down's syndrome, Tourette's syndrome, neurosis during chronic cerebral infarction stage, cerebral dysfunction caused by cerebral injury, anxiety, schizophrenia, depression, Huntington's disease, pain and so on.

The compounds of formula (I) or a pharmaceutically acceptable salt thereof according to the present invention may be administered in the form of oral or parenteral formulations. The formulations for oral administration may include for example, tablets, capsules, granules, fine powders, syrups or the like; the formulations for parenteral administration may include, for example, injectable solutions or suspensions with distilled water for injection or other pharmaceutically acceptable solution, patches for transdermal application, sprays for nasally administration, depositories or the like.

These formulations may be formed by mixing with pharmaceutically acceptable carrier, excipient, sweeter, stabilizer and so on by the conventional procedures known *per se* to those skilled in the field of pharmaceutical formulations.

Examples of pharmaceutically acceptable carrier or excipient include polyvinyl pyrrolidone, gum arabic, gelatin, sorbit, cyclodextrin, magnesium stearate, talc, polyethylene glycol, polyvinyl alcohol, silica, lactose, crystalline cellulose, sugar, starch, calcium phosphate, vegetable oil, carboxymethylcellulose, hydroxypropylcellulose, sodium lauryl sulfate, water, ethanol, glycerol, mannitol, syrup and the like.

Examples of solution for injection include isotonic solution containing glucose and the like, and these solutions can further contain an appropriate solubilizer such as polyethylene glycol or the like, buffer, stabilizer, preservative, antioxidant and so on.

These formulations can be administered to the human being and other mammalian animals, and the preferable administration route may include oral route, transdermic route, nasal route, rectal route, topical route or the like.

Administration dose may vary in a wide range with ages, weights, condition of patients, routes of administration or the like, and a usual recommended daily dose to adult patients for oral administration is within the range of approximately 0.001-1,000 mg/kg per body weight, preferably 0.01-100 mg/kg per body weight, and more preferably 0.1-10 mg/kg per body weight. In the case of parenteral administration such as intravenous injections, a usual recommended daily dose is within the range of approximately 0.00001-10 mg/kg per body weight, preferably 0.0001-1 mg/kg per body weight, and more preferably 0.001-0.1 mg/kg per body weight, once or in three times per day.

Methods for evaluating binding capabilities of the compounds at nicotinic acetylcholine receptors are different by subtypes of receptors. Binding capabilities of the compounds at α4β2 nicotinic acetylcholine receptors are examined using rat brain membrane obtained from whole homogenized brain, and determining the inhibiting rate of the compounds against [³H]-cytisine binding to said brain membrane. Furthermore, the binding capabilities of the compounds at α1β1γδ nicotinic acetylcholine receptors are examined using homogenized rat muscle, and determining the inhibiting rate of the compounds against [³H]-α-bungarotoxin binding to said muscle homogenate.

Agonist effect in human α4β2 subtype of nicotinic acetylcholine receptors are examined by using human nicotinic acetylcholine receptors prepared in oocytes of *Xenopus laevis*, which is injected with cRNA from the corresponding cloning cDNA of human α4 and β2 subunits of nicotinic acetylcholine receptors, and to measure the expression of electric response by adding the test compounds to perfusion solution by means of membrane potential holding method.

### Examples:

The present invention is illustrated in more detail by way of the following examples.

### Example 1: Synthesis by the Process 1

### 2-(6-Chloro-3-pyridyl)methyl-3-imino-6-phenyl-2,3-dihydro-pyridazine [Compound 44]

300 mg (1.5 mmol) of 2-chloro-5-chloromethylpyridine hydrochloride was dissolved in dichloromethane and the saturated aqueous solution of sodium hydrogencarbonate was mixed to separate into organic layers. The resultant organic layer was dried with potassium carbonate and the solvent was removed off under reduced pressure. The resultant oily residue and 171 mg (1 mmol) of 3-amino-6-phenylpyridazine were dissolved in 5 ml of N,N-dimethylformamide and the reaction mixture was heated at 80°C for 8 hours. Then, the reaction mixture was cooled to the room temperature, and diluted with 2-propanol. The resultant crystals were collected by filtration and dried under reduced pressure to give 243 mg (yield: 73%) of hydrochloride of the title Compound 44.

The following compounds were synthesized in accordance with the procedures as described in Example 1.
Compound 1: 2-imino-3-(3-pyridyl)methyl-2,3-dihydrothiazole;
Compound 2: 3-(6-chloro-3-pyridyl)methyl-2-imino-4-methyl-2,3-dihydrothiazole;
Compound 3: 3-(6-chloro-3-pyridyl)methyl-2-imino-5-methyl-2,3-dihydrothiazole;
Compound 4: 2-imino-3-(3-pyridyl)methylthiazolidine;
Compound 5: 3-(6-chloro-3-pyridyl)methyl-2-iminothiazolidine;
Compound 6: 6-chloro-2-(6-chloro-3-pyridyl)methyl-3-imino-2,3-dihydropyridazine;
Compound 7: 1-(6-chloro-3-pyridyl)methyl-2-imino-1,2-dihydropyridine;
Compound 8: 3-(6-chloro-3-pyridyl)methyl-2-imino-2,3-dihydrothiazole;
Compound 9: 2-amino-1-(6-chloro-3-pyridyl)methylimidazole;
Compound 10: 1-(6-chloro-3-pyridyl)methyl-2-imino-1,2-dihydropyrimidine;
Compound 11: 3-(6-bromo-3-pyridyl)methyl-2-imino-2,3-dihydrothiazole;
Compound 12: 3-(6-fluoro-3-pyridyl)methyl-2-imino-2,3-dihydrothiazole;
Compound 16: 3-(6-chloro-3-pyridyl)methyl-2-imino-3,4,5,6-tetrahydro-2H-1,3-oxazine;
Compound 17: 3-(6-chloro-3-pyridyl)methyl-2-imino-3,4,5,6-tetrahydro-2H-1,3-thiazine;
Compound 18: 3-(6-fluoro-3-pyridyl)methyl-2-imino-4-methyl-2,3-dihydrothiazole;
Compound 19: 3-(6-bromo-3-pyridyl)methyl-2-imino-4-methyl-2,3-dihydrothiazole;
Compound 20: 3-(6-chloro-3-pyridyl)methyl-2-imino-4,5-dimethyl-2,3-dihydrothiazole;
Compound 21: 3-(6-chloro-3-pyridyl)methyl-4-ethyl-2-imino-2,3-dihydrothiazole;
Compound 22: 5-chloro-1-(6-chloro-3-pyridyl)methyl-2-imino-1,2-dihydropyridine;
Compound 23: 1-(6-chloro-3-pyridyl)methyl-2-imino-3-methyl-1,2-dihydropyridine;
Compound 24: 1-(6-chloro-3-pyridyl)methyl-2-imino-5-methyl-1,2-dihydropyridine;
Compound 25: 1-(6-chloro-3-pyridyl)methyl-2-imino-4-methyl-1,2-dihydropyridine;
Compound 26: 2-imino-1-(3-pyridyl)methyl-1,2-dihydropyridine;
Compound 27: 3-(6-chloro-3-pyridyl)methyl-2-imino-4-methylthiazolidine;
Compound 28: 3-(6-chloro-3-pyridyl)methyl-2-iminooxazolidine;
Compound 30: 3-(5-bromo-3-pyridyl)methyl-2-imino-4-methyl-2,3-dihydrothiazole;
Compound 31: 3-(4-chlorobenzyl)-2-iminothiazolidine;
Compound 32: 2-imino-3-(6-methyl-3-pyridyl)methylthiazolidine;
Compound 33: 2-imino-3-(4-pyridazinyl)methylthiazolidine;
Compound 34: 3-(2-chloro-5-thiazolyl)methyl-2-iminothiazolidine;
Compound 35: 2-imino-3-(3-methyl-5-isoxazolyl)methylthiazolidine;
Compound 36: 2-imino-4-methyl-3-(3-methyl-5-isoxazolyl)methyl-2,3-dihydrothiazole;
Compound 37: 3-(2-chloro-5-thiazolyl)methyl-2-imino-4-methyl-2,3-dihydrothiazole;
Compound 38: 3-(5,6-dichloro-3-pyridyl)methyl-2-imino-4-methyl-2,3-dihydrothiazole;
Compound 39: 2-imino-4-methyl-3-(6-methyl-3-pyridyl)methyl-2,3-dihydrothiazole;
Compound 40: 3-(6-chloro-3-pyridyl)methyl-2-Lmlno-5-phenyl-2,3-dihydrothiazole;
Compound 41: 3-(6-chloro-3-pyridyl)methyl-2-imino-4-phenyl-2,3-dihydrothiazole;
Compound 42: 4-(4-chlorophenyl)-3-(6-chloro-3-pyridyl)methyl-2-imino-2,3-dihydrothiazole;
Compound 43: 3-(6-chloro-3-pyridyl)methyl-2-imino-4-phenylthiazolidine;
Compound 44: 2-(6-chloro-3-pyridyl)methyl-3-imino-6-phenyl-2,3-dihydropyridazine;
Compound 45: 3-imino-6-phenyl-2-(3-pyridyl)methyl-2,3-dihydropyridazine;
Compound 46: 1-(6-chloro-3-pyridyl)methyl-2-imino-5-phenyl-1,2-dihydropyrimidine;
Compound 47: 1-(6-chloro-3-pyridyl)methyl-2-imino-5-nitro-1,2-dihydropyridine;
Compound 48: 2-imino-1-(6-methyl-3-pyridyl)methyl-1,2-dihydropyridine;
Compound 49: 2-imino-3-(3-pyridazinyl)methylthiazolidine;
Compound 50: 2-amino-1-(2-chloro-5-thiazolyl)methylimidazole;
Compound 51: 2-amino-1-(6-chloro-3-pyridyl)methyl-4,5-dimethylimidazole;
Compound 52: 2-amino-1-(5-pyrimidyl)methylimidazole;
Compound 53: 2-amino-1-(6-chloro-3-pyridyl)methyl-4-methylimidazole;
Compound 54: 2-amino-1-(5,6-dichloro-3-pyridyl)methylimidazole;
Compound 55: 2-amino-1-(3-pyridyl)methylimidazole;
Compound 56: 2-amino-1-(6-methyl-3-pyridyl)methylimidazole;
Compound 57: 3-(4-chlorobenzyl)-2-imino-2,3-dihydrothiazole;
Compound 58: 2-amino-1-(4-chlorobenzyl)imidazole;
Compound 59: 2-amino-1-(7-aza-3-indolyl)methylimidazole;
Compound 60: 3-(3,4-dichlorobenzyl)-2-imino-2,3-dihydrothiazole;
Compound 61: 2-imino-3-(3-nitrobenzyl)-2,3-dihydrothiazole;
Compound 62: 2-imino-3-(4-nitrobenzyl)-2,3-dihydrothiazole;
Compound 63: 2-imino-3-(4-methylbenzyl)-2,3-dihydrothiazole;
Compound 64: 2-imino-3-(3-trifluoromethylbenzyl)-2,3-dihydrothiazole;
Compound 65: 3-(4-cyanobenzyl)-2-imino-2,3-dihydrothiazole;
Compound 66: 3-(7-aza-3-indolyl)-2-imino-2,3-dihydrothiazole;

### Example 2: Synthesis by the Process 2

### 1-(6-Chloro-3-pyridyl)methyl-2-iminoimidazolidine [Compound 13]

To a suspension of 335 mg (1.3 mmol) of 1-(6-chloro-3-pyridyl)methyl-2-nitroiminoimidazolidine in 20 ml of methanol were added 6 ml of 20% titanium (III) chloride, and the mixture was stirred at room temperature for 1 hour and 20 minutes under nitrogen gas atmosphere. Then, the solvent was removed under reduced pressure, and 50% sodium hydroxide aqueous solution was added to the resulting residue under ice-cooling. The insoluble matter was removed off by filtration using Celite, and the filtrate was concentrated under reduced pressure. To the resulting residue was added dichloromethane and methanol (20:1) mixture solvent, insoluble matter was removed off by filtration, and the filtrate was concentrated under reduced pressure. The resulting residue was purified by aminopropyl-coated silica gel (Chromatorex NH-type; Fuji Silysia Chemical Ltd.) column chromatography (eluent; dichloromethane : methanol = 20:1) to give 182 mg (yield; 66%) of 1-(6-chloro-3-pyridyl)methyl-2-iminoimidazolidine as colorless crystalline product. This product was dissolved in methanol and to this solution was added 100 mg (0.862 mmol) of fumaric acid, and the mixture was concentrated under reduced pressure. The resulting crystalline residue was treated with acetonitrile, filtrated and dried *in vacuo* to give 222 mg of fumarate of the title Compound 13.

### Example 3: Synthesis by the Process 3

### 1-(6-Chloro-3-pyridyl)methyl-2-iminopyrrolidine [Compound 14]

A mixture of 713 mg (5 mmol) of (6-chloro-3-pyridyl)methylamine, 745 mg (5 mmol) of 4-bromobutyronitrile, and 1.04 g (7.5 mmol) of potassium carbonate in 15 ml of N,N-dimethylformamide was stirred at room temperature for 17 hours. Then, the solvent was removed under reduced pressure and the resulting residue was mixed with dichloromethane and water, and the organic layer was separated. The organic layer was dried over magnesium sulfate, and the solvent was removed under reduced pressure. The resulting residue was purified by aminopropyl-coated silica gel (Chromatorex NH-type; Fuji Silysia Chemical Ltd.) column chromatography (eluent; n-hexane : ethyl acetate = 3:1) to give 505 mg (yield; 48%) of 4-(6-chloro-3-pyridyl)methylamino-butyronitrile as colorless oil. 500 mg (2.38 mmol) of 4-(6-chloro-3-pyridyl)methylaminobutyronitrile was dissolved in 15 ml of toluene under argon gas atmosphere, and 2.6 ml of 1M trimethylaluminum/n-hexane solution was added. The mixture was heated at 90°C for 14 hours under refluxing. After the reaction, the reaction mixture was cooled to the room temperature and to this mixture was added 10 ml of chloroform, 5 ml of methanol, and 1 ml of water in order, and the resulting gel was removed off by filtration. The filtrate was condensed under reduced pressure, and the residue was purified by aminopropyl-coated silica gel (Chromatorex NH-type; Fuji Silysia Chemical Ltd.) column chromatography (eluent; dichloromethane : methanol = 50:1) to give 452 mg (yield; 90%) of 1-(6-chloro-3-pyridyl)methyl-2-iminopyrrolidine as yellow oil. Part of this product i.e., 210 mg (1 mmol) of this product was dissolved in methanol and to this solution was added 116 mg (1 mmol) of fumaric acid, and the mixture was concentrated under reduced pressure. The resulting oily residue was treated with acetonitrile to crystallize. The crystals were collected by filtration and dried *in vacuo* to give 309 mg of fumarate of the title Compound 14.

The compound 15: 1-(6-chloro-3-pyridyl)methyl-2-imino-piperidine was synthesized according to this Example 3.

### Example 4: Synthesis by the Process 4

### 1-(6-Chloro-3-pyridyl)methyl-2-imino-1,2,3,4,5,6-hexahydro-pyrimidine [Compound 29]

A mixture of 237 mg (1 mmol) of N-(3-aminopropyl)-N-[(6-chloro-3-pyridyl)methyl]amine hydrochloride and 303 mg (2.5 mmol) of dithiocarbimidoic acid dimethyl ester in 5 ml of N,N-dimethylformamide was stirred at 90 °C for 1 hour and 50 minutes. Then, the solvent was removed off under reduced pressure and the resulting residue was purified by aminopropyl-coated silica gel (Chromatorex NH-type; Fuji Silysia Chemical Ltd.) column chromatography (eluent; from dichloromethane to dichloromethane : methanol = 9:1) to give 77 mg (yield; 34%) of 1-(6-chloro-3-pyridyl)methyl-2-imino-1,2,3,4,5,6-hexahydropyrimidine as colorless oil. The resultant oil was dissolved in 5 ml of methanol and to this solution was added 0.01 ml of 4M-hydrogen chloride/dioxane, and the mixture was stirred at room temperature for 5 minutes, and concentrated under reduced pressure. The resulting oily residue was treated with acetone to crystallize. The crystals were collected by filtration and dried *in vacuo* to give 14 mg of dihydrochloride of the title Compound 29.

Physicochemical data of the Compound 1 to Compound 66 obtained by above-mentioned examples are summarized in the following Table 1 to Table 14.

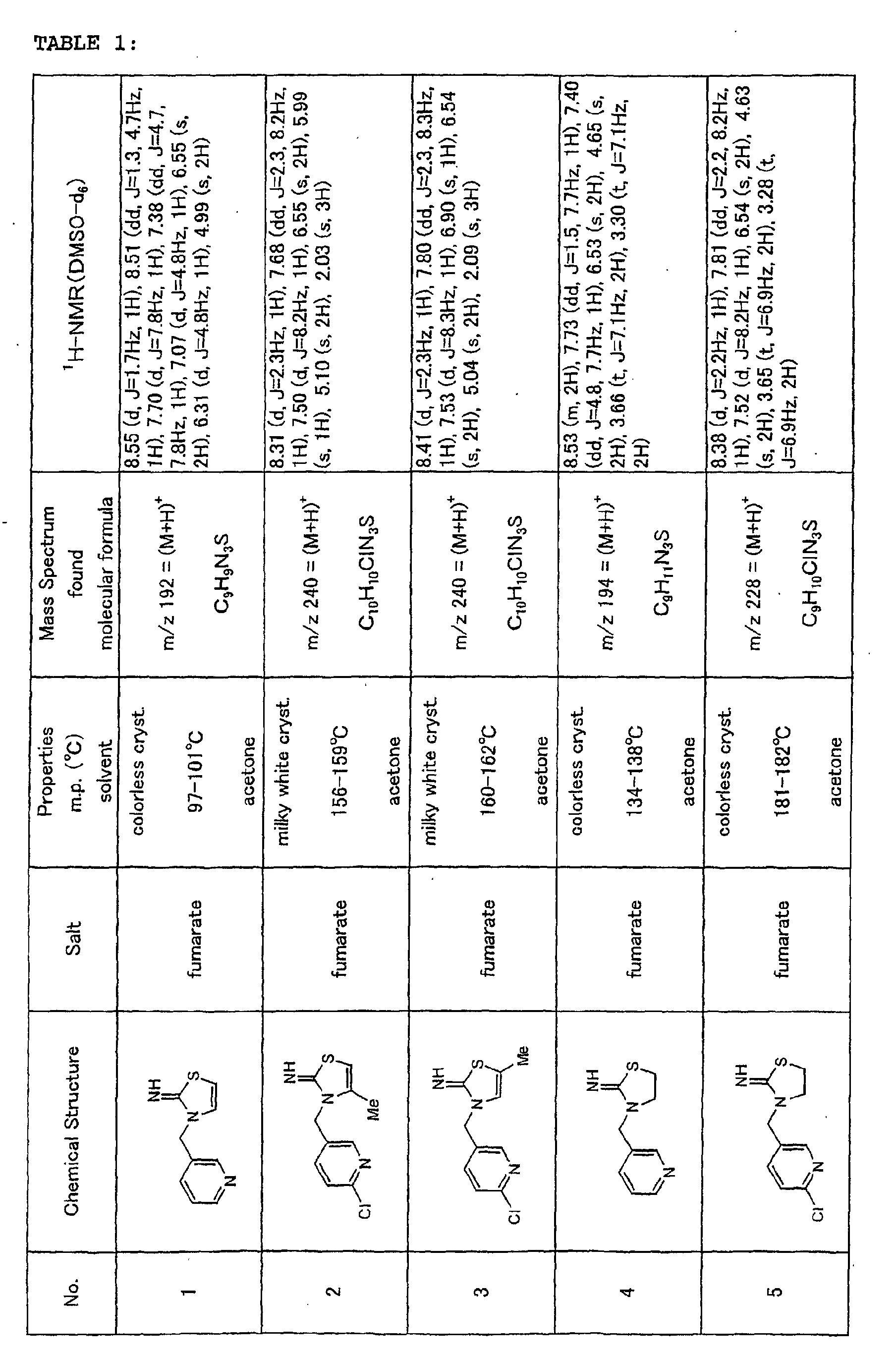

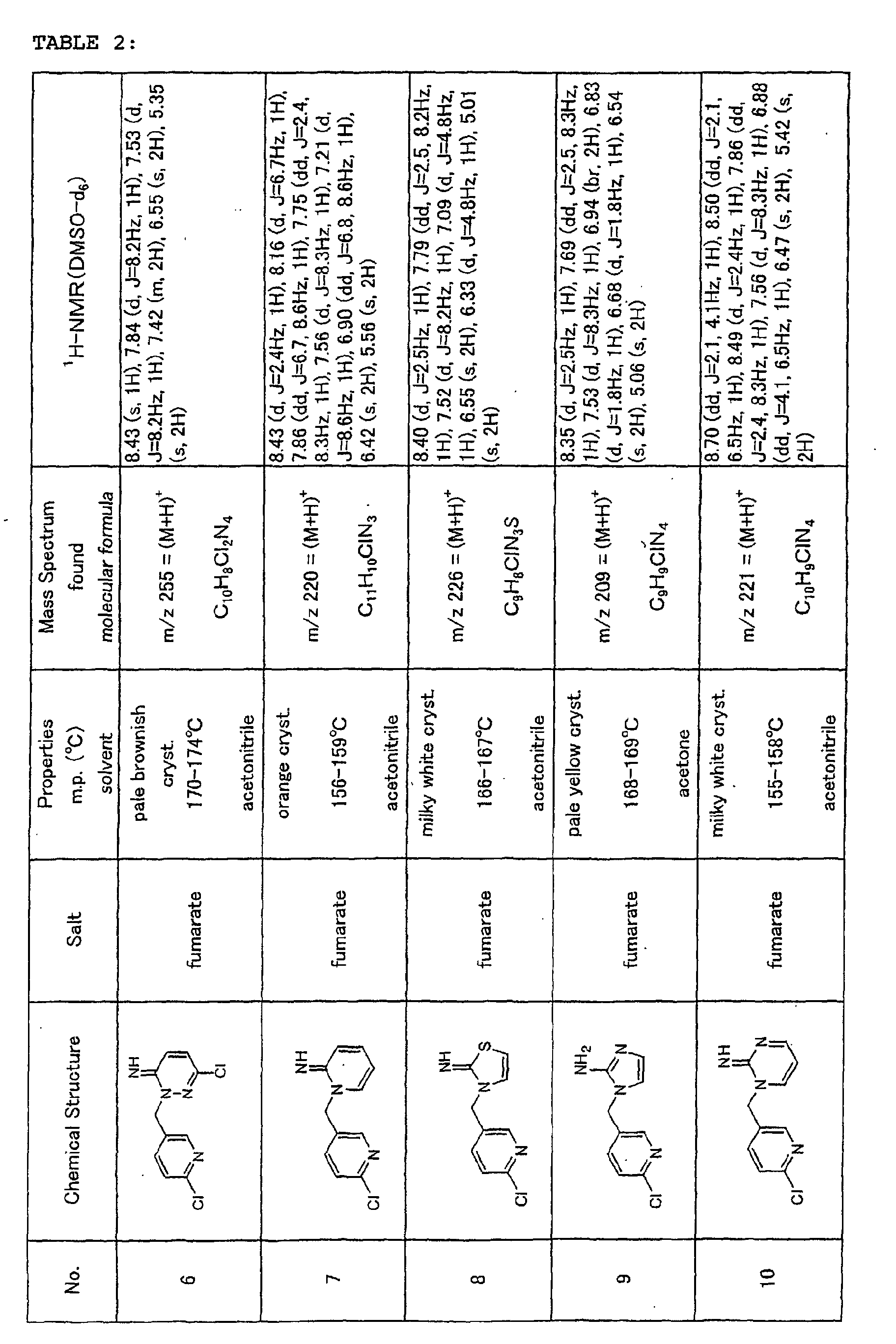

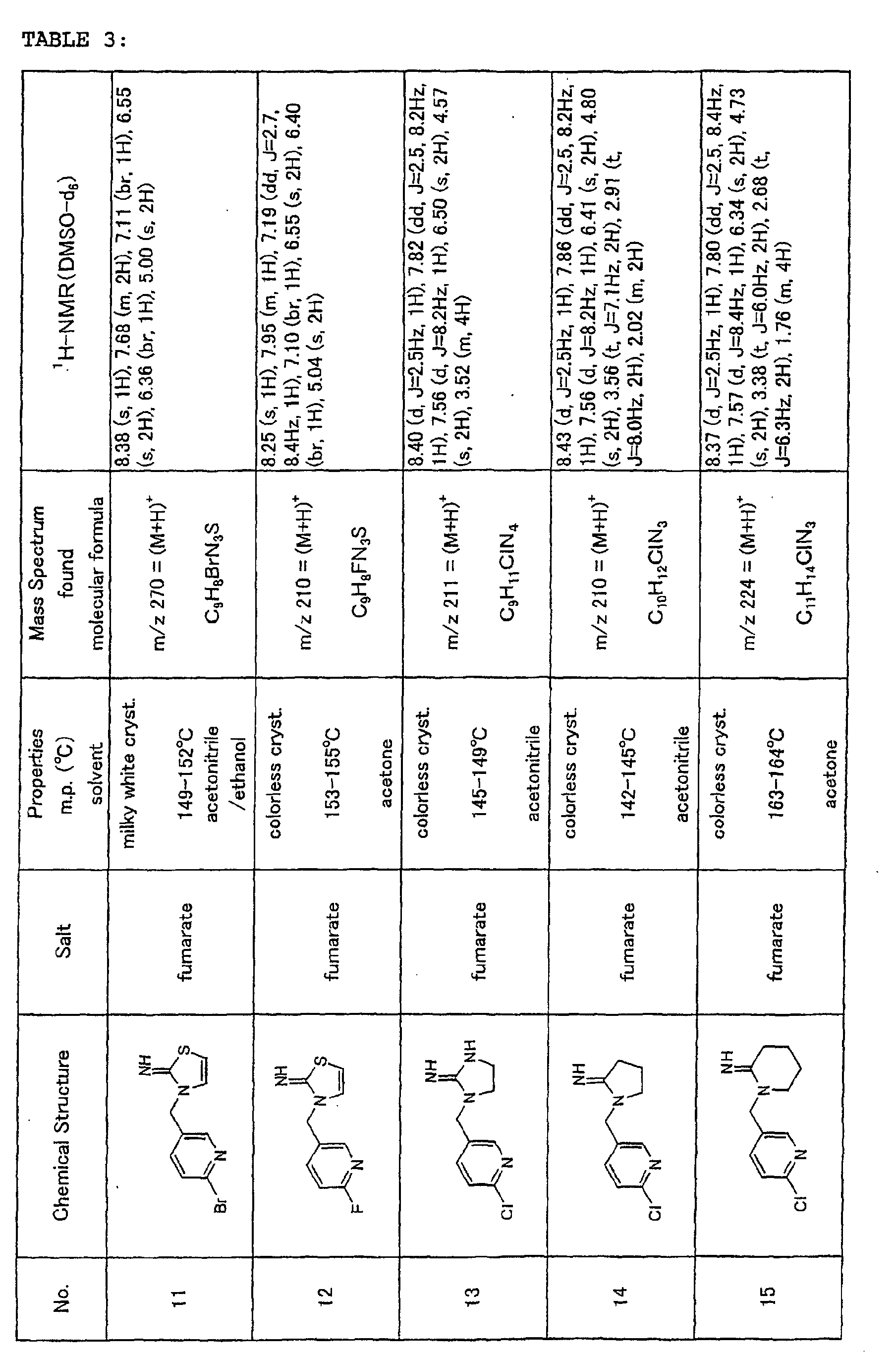

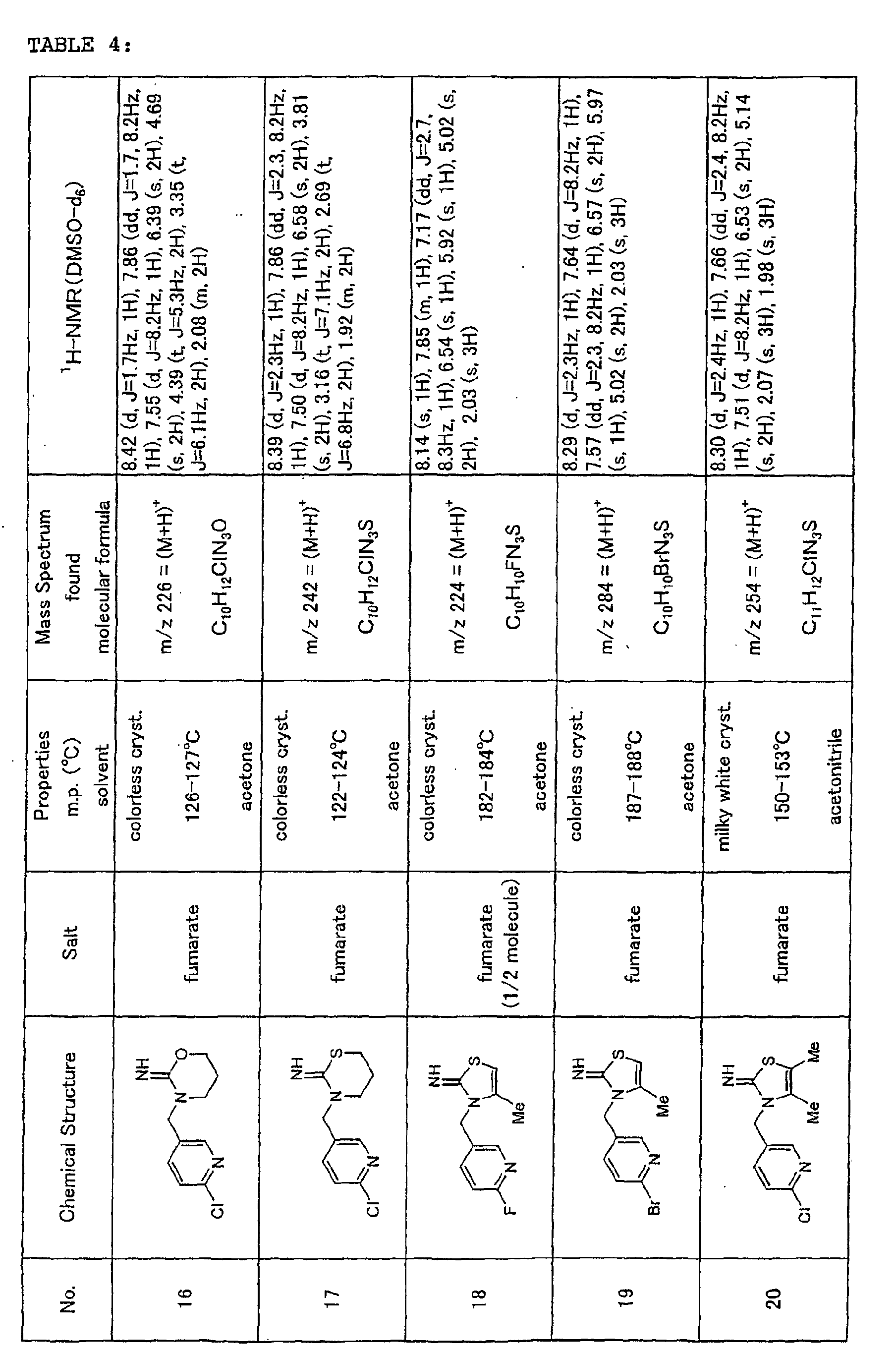

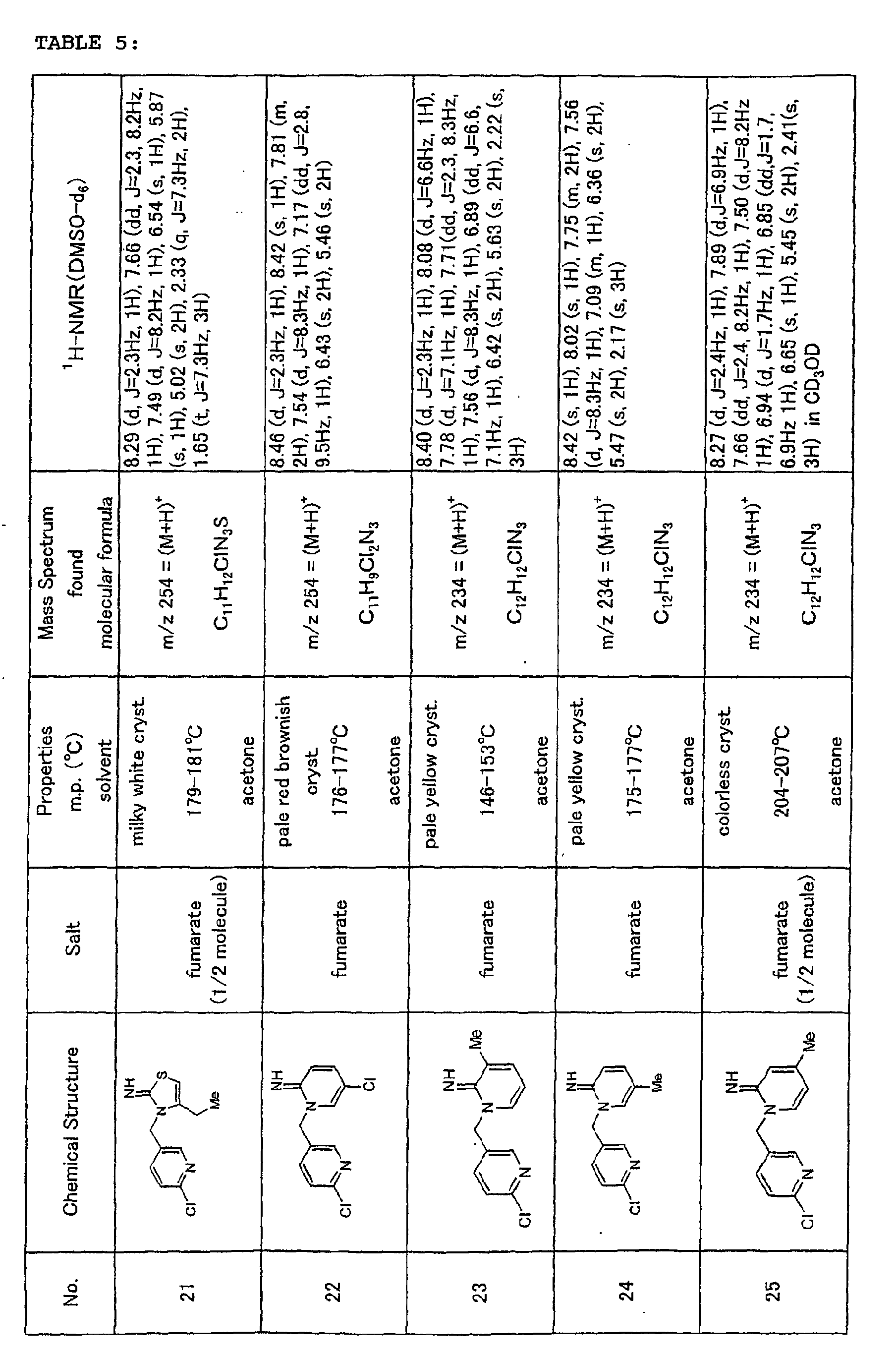

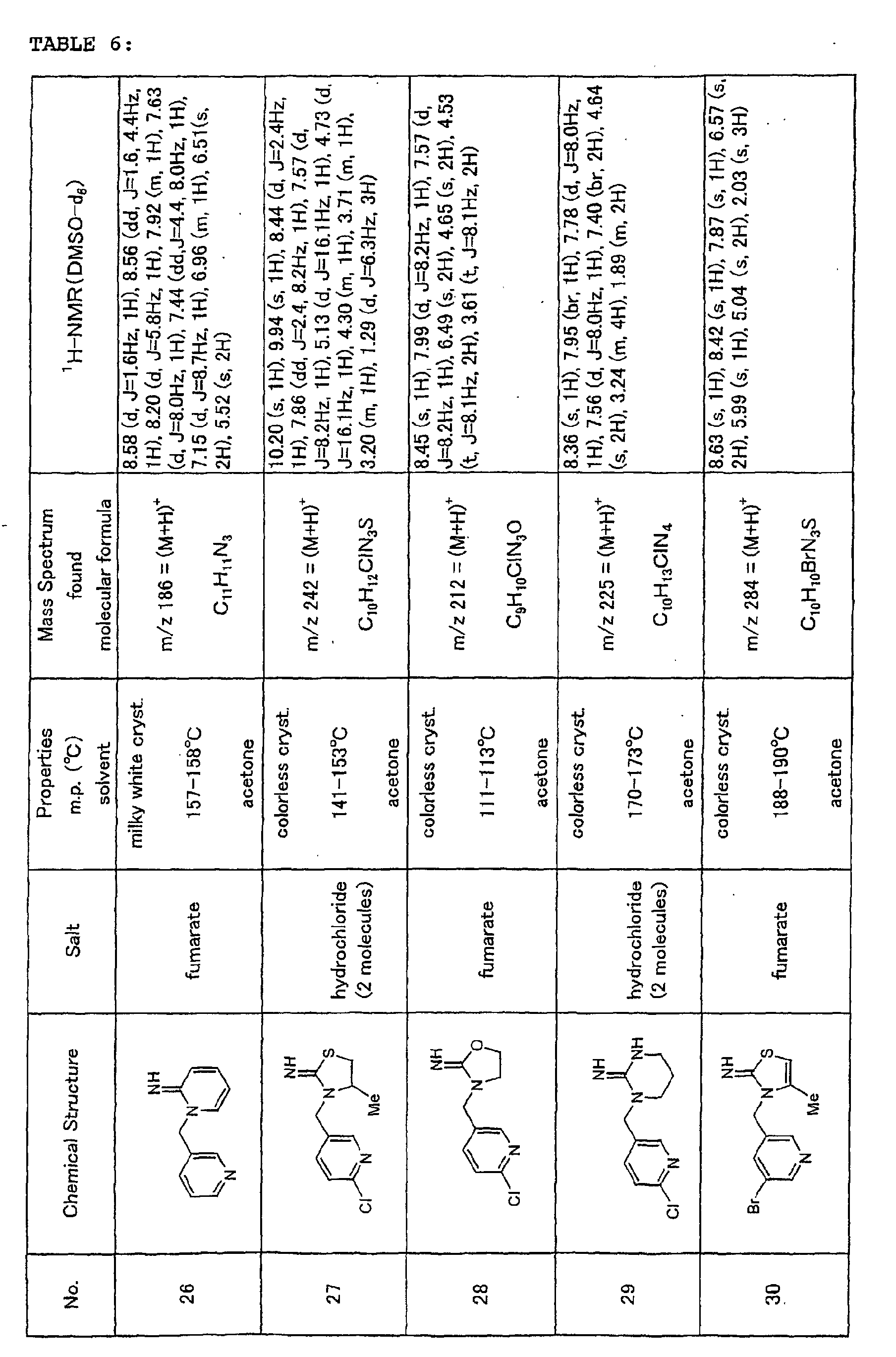

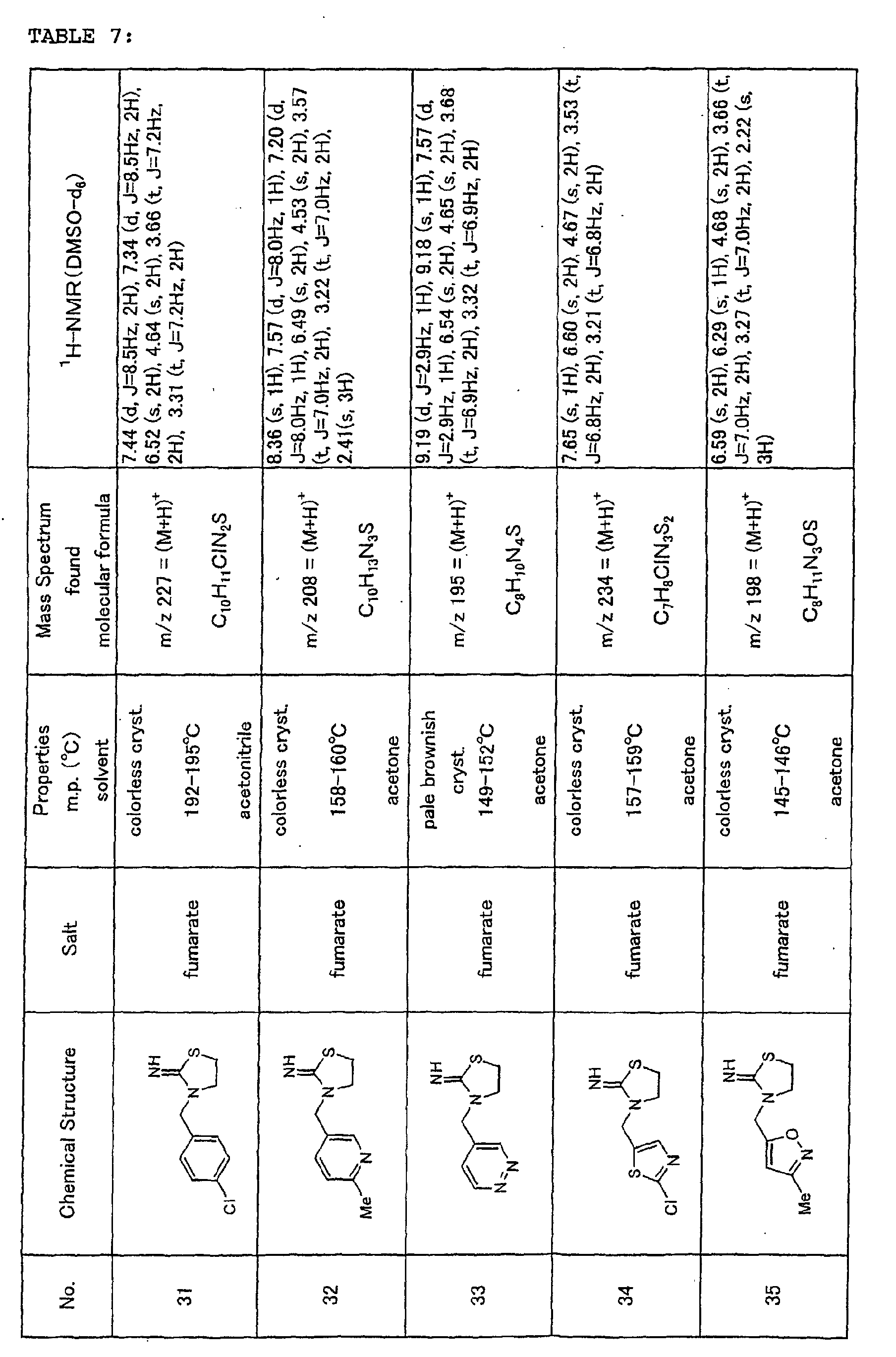

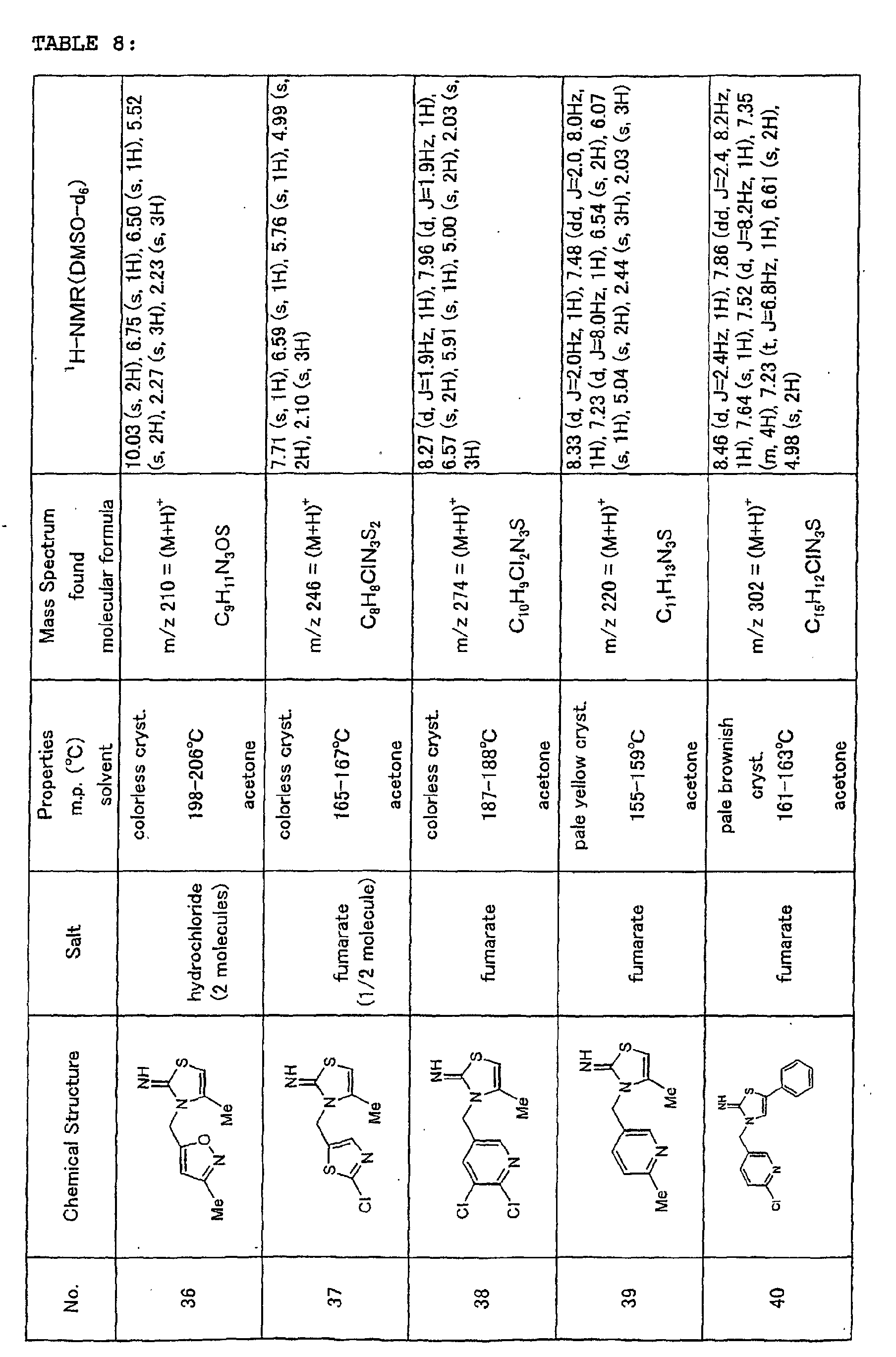

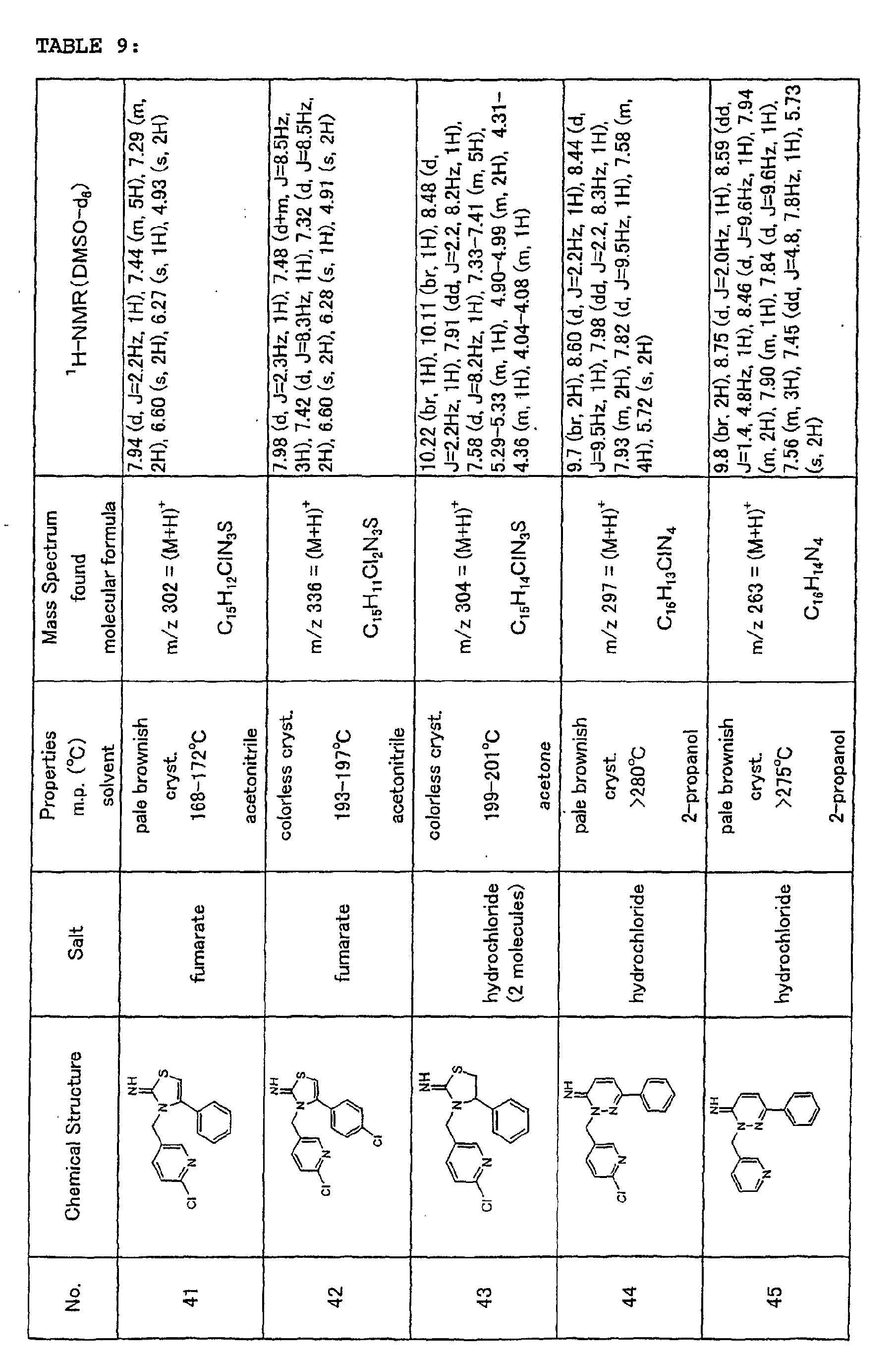

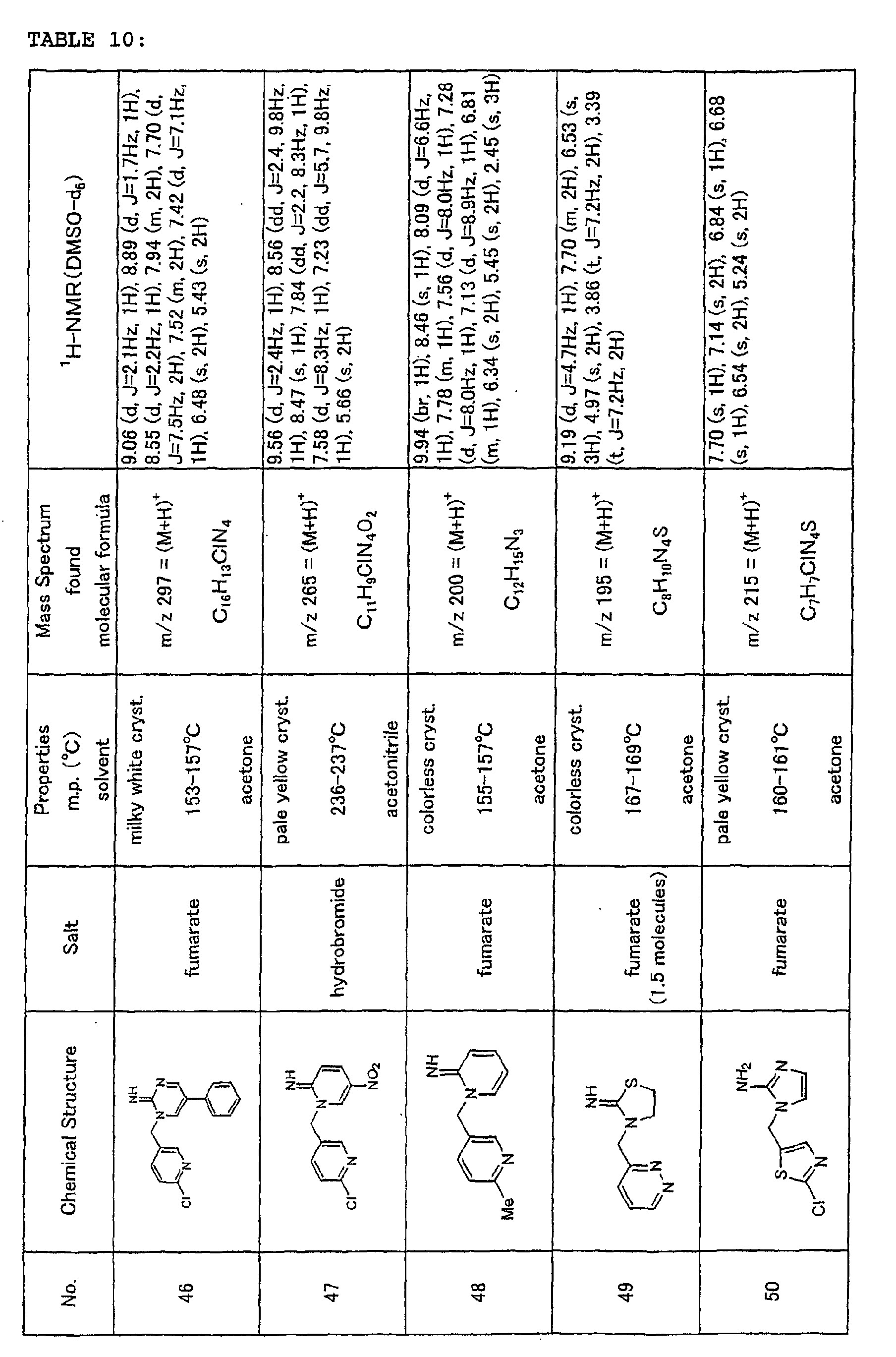

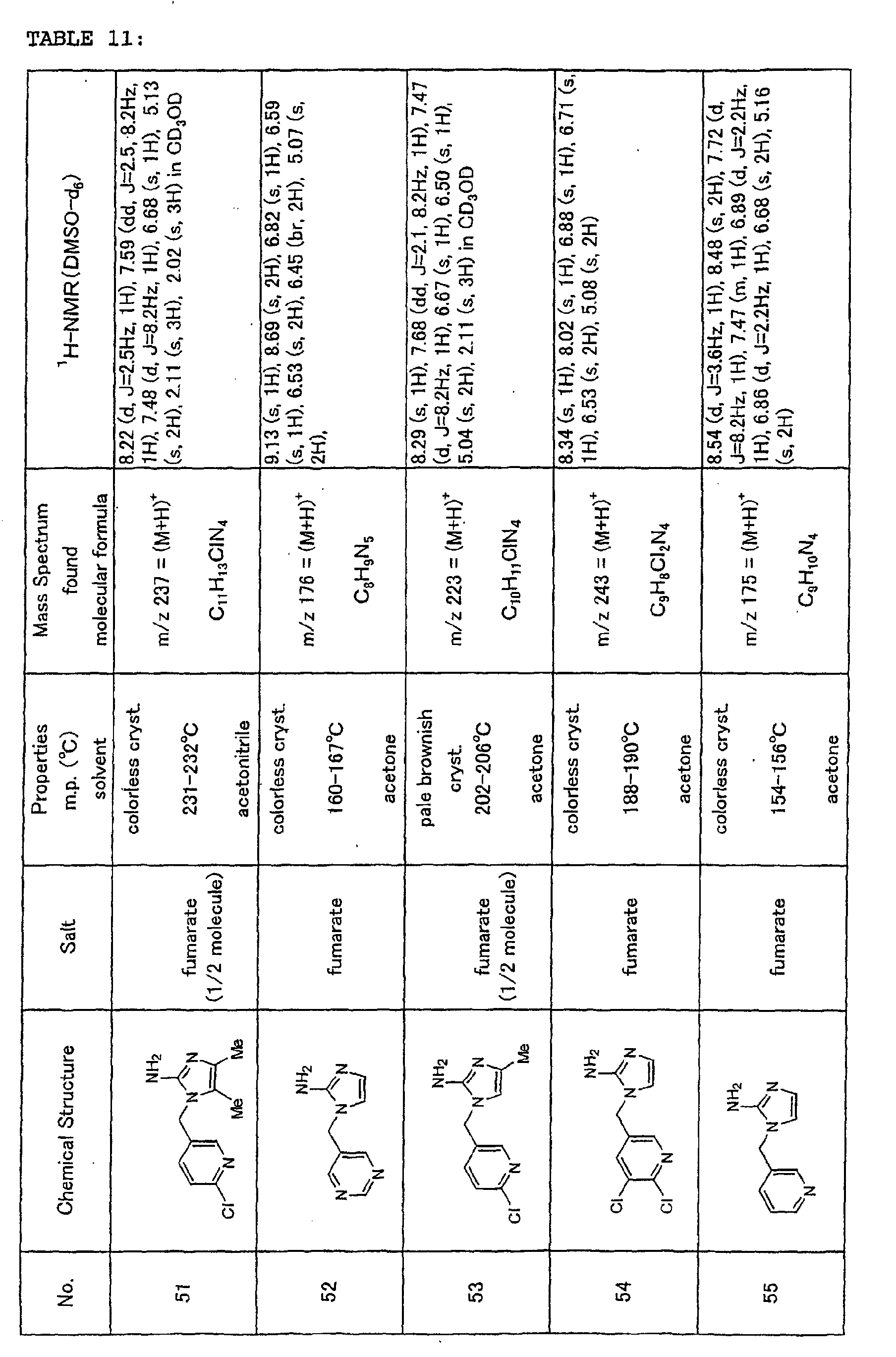

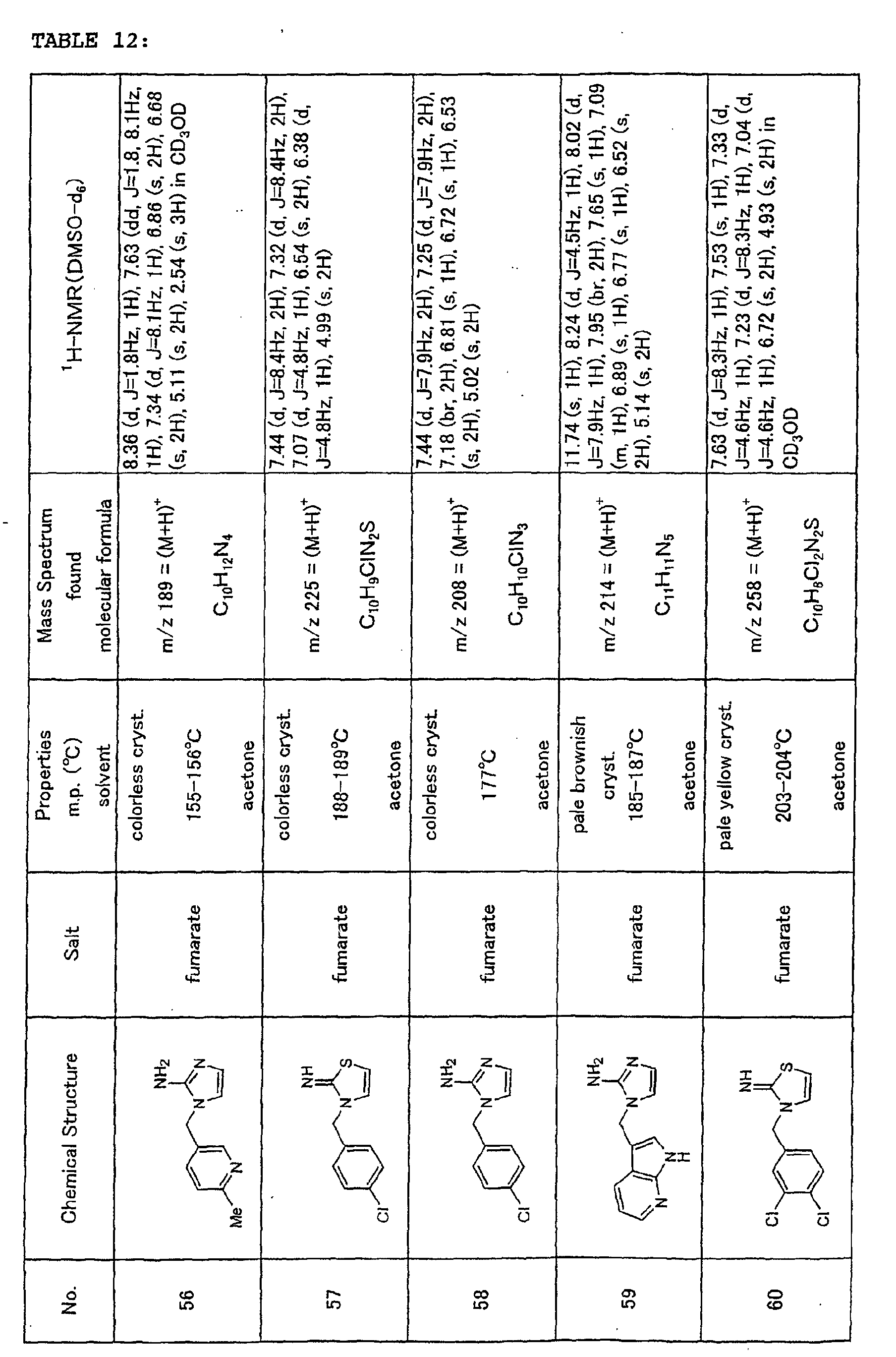

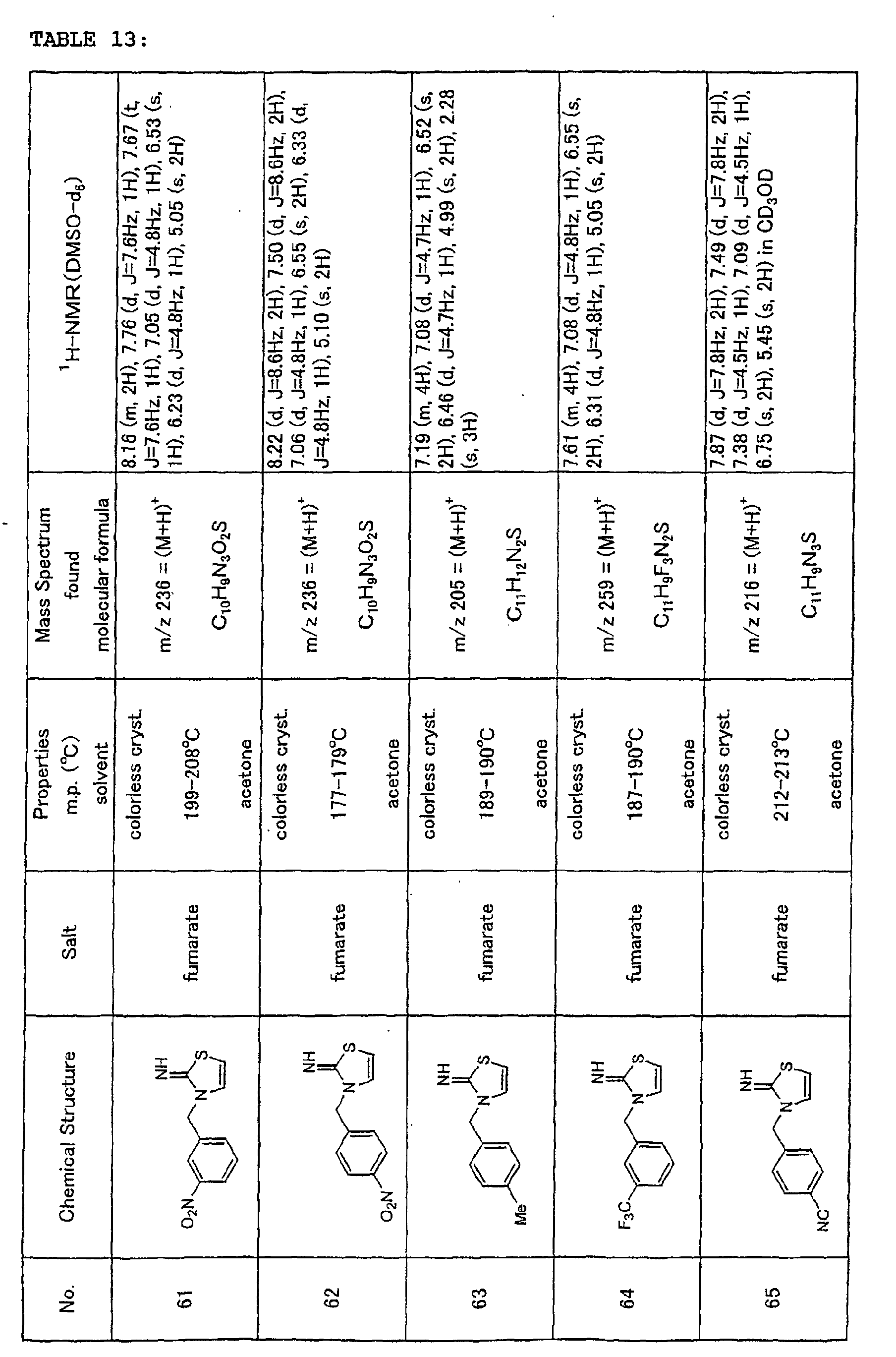

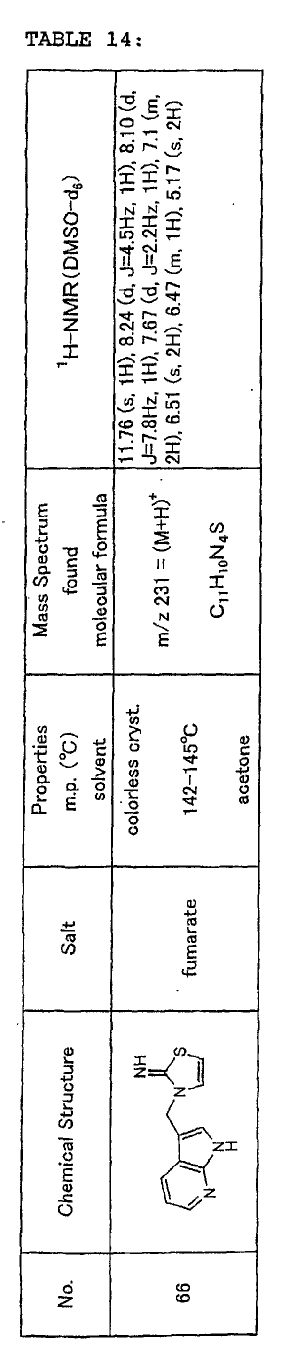

Effect of the compounds (I) of the present invention was evaluated by the following biological experiments.

### Biological Experiment 1:

### Binding assays at α4β2 subtype of nicotinic acetylcholine receptors

Affinity of the compounds of the present invention to α4β2 subtype of nicotinic acetylcholine receptors was performed by the following method, which was modified method described by Pabreza L. A., Dhawan S. & Kellar K. *J., Mol*. *Pharm*., 39, 9-12 (1990), and by Anderson D. J. & Arneric S. P., *Eur*. *J. Pharm*., 253, 261-267 (1994).

### (1) Preparation of rat brain membrane containing α4β2 subtype of nicotinic acetylcholine receptors

Fischer-344 strain male rats (body weight: 200-240 g; 9 weeks old) obtained from Charles River Japan were used. Rats were housed in the breeding cage controlled of the room temperature at 23 ± 1°C, and the humidity of 55 ± 5% for 1 to 4 weeks. Rats (3 to 4 rats per a cage) were housed with lights on for 12 hours daily (from 7:00 to 19:00), and allowed free access to food and water.

Preparation of rat brain membrane containing α4β2 subtype of nicotinic acetylcholine receptors was performed as follow. That is, rat brains were isolated just after sacrificed by decapitation, washed with ice-cooled saline solution and then frozen at -80°C with liquid nitrogen and stored till using. After thawing the frozen brain, the brain was homogenized in 10 volumes of ice-cooled buffer solution (50 mM of Tris-HCl, 120 mM of NaCl, 5 mM of KCl, 1 mM of MgCl₂, 2mM of CaCl₂; pH 7.4; 4°C) using homogenizer (HG30, Hitachi Kohki Ltd.) for 30 seconds, and the homogenate were centrifuged under 1,000 x G for 10 minutes at 4°C. The resulting supernatant was separated and the pellet was homogenized again with half volume of aforementioned prior buffer solution and centrifuged under the same conditions. Combined supernatant was further centrifuged under 40,000 x G for 20 minutes at 4°C. The pellet was suspended in buffer solution and used for binding assays at receptors.

### (2) Experiments of α4β2 subtype of nicotinic acetylcholine receptors binding

Suspensions of membrane pellets containing 400-600 µg of protein were added to test tubes containing test compounds and [³H]-cytisine (2 nM) in a final volume of 200 µl and incubated for 75 minutes in ice-cooled bath. The samples were isolated by vacuum filtration onto Whatman GF/B filters, which were prerinsed with 0.5% polyethylenimine just prior to sample filtration, using Brandel multi manifold cell harvester. The filters were rapidly washed with buffer solution (3 x 1 ml). The filters were counted in 3 ml of clearsol I (Nacalai Tesque Inc.). The determination of nonspecific binding was incubated in the presence of 10 µM (-)-nicotine.

Analyses of the experimental results were conducted using the Accufit Competition Program (Beckman Ltd.).

### Biological Experiment 2:

### Binding assays at α1β1γδ subtype of nicotinic acetylcholine receptors

Affinity of the compounds of the present invention to α1β1γδ subtype of nicotinic acetylcholine receptors was measured by the following method, which was modified method described by Garcha H. S., Thomas P., Spivak C. E., Wonnacott S. & Stolerman I. *P., Psychropharmacology*, 110, 347-354 (1993).

### (1) Preparation of rat skeletal muscles containing α1β1γδ subtype of nicotinic acetylcholine receptors

The substantially same animals described in the Biological Experiment 1 were used.

Isolation of α1β1γδ subtype of nicotinic acetylcholine receptors was performed as follow. That is, rat posterior skeletal muscles were isolated just after sacrificed by decapitation, washed with ice-cooled saline solution and then frozen at -80°C with liquid nitrogen and stored till using. After thawing the frozen muscles, tissue was homogenized (40% w/v) with buffer solution [2.5 mM of sodium phosphate buffer (pH:7.2), 90 mM of NaCl, 2 mM of KCl, 1 mM of EDTA, 2 mM of benzamidine, 0.1 mM of benzethonium chloride, 0.1 mM of PMSF, 0.01% of sodium azide] in Waring blender (Waring blender 34BL97; WARING PRODUCTS DIVISION DYNAMICS CORPORATION OF AMERICA) for 60 seconds. The homogenate were centrifuged under 20,000 x G for 60 minutes at 4°C. The supernatant was separated and the resulting pellet was added to the same buffer (1.5 ml/g wet weight), and homogenized under the same conditions. Triton X100 (2% w/v) was added and the mixture was stirred for 3 hours at 4°C. The centrifugation at 100,000 x G for 60 minutes at 4°C yielded the rat muscle extract as supernatant. This was stored at 4°C for up to 4 weeks, and used for binding assays at receptors.

### (2) Experiments of α1β1γδ subtype of nicotinic acetylcholine receptors binding

Receptors binding experiments were performed as follow. That is, the extract of rat muscle containing 600-900 µg of protein was added to test tubes containing test compounds and incubated for 15 minutes at 37°C. Then, to this mixture was added 1 nM of [³H]-α-bungarotoxin (α-Bgt) and further incubated for 2 hours. The samples were isolated by vacuum filtration onto Whatman GF/B filters, which were prerinsed with 0.5% polyethylenimine just prior to sample filtration, using Brandel multi manifold cell harvester. The filters were rapidly rinsed with washing solution (10 mM of KH₂PO₄, 150 mM of NaCl, pH 7.2, room temperature) (5 x 1 ml). The filters were counted in 3 ml of clearsol I (Nacalai Tesque Inc.). Determination of nonspecific binding was incubated in the presence of 1 µM α-Bgt.
The solutions containing α-Bgt (labeled/non-labeled) were prepared by using buffer solution containing 0.25% of BSA. In the receptor binding experiments, said buffer solution was added for adjusting the final concentration of BSA to be 0.05%.

Analyses of the experimental results were conducted by the same way as described in the Biological Experiment 1.

Table 15, 16 and 17 show the results of receptor binding studies of the compounds of the present invention and (-) - nicotine as reference compound.

**TABLE 15:**

| Compound No. | Affinities for receptors Ki | |
|---|---|---|
| | α4β2 ^{*1} | α1β1γδ ^{**2} |
| 1 | 4.84 nM | 4.9 µM |
| 2 | 3.5 nM | 12.8 µM |
| 3 | 5.8 nM | (69%, 28%) |
| 4 | 7.5 nM | (6%, 1%) |
| 5 | 2.2 nM | 7.65 µM |
| 6 | 15 nM | (44%, 15%) |
| 7 | 3.1 nM | 71.2 µM |
| 8 | 0.5 nM | 10.2 µM |
| 9 | 22.2 nM | (86%, 49%) |
| 10 | 8.7 nM | 347 µM |
| 11 | 0.63 nM | (13%, 5%) |
| 12 | 1.89 nM | (20%, -2%) |
| 13 | 4.6 nM | (26%, 8%) |
| 14 | 1.9 nM | (14%, 0%) |
| 15 | 4.8 nM | (21%, 4%) |
| 16 | 0.65 nM | (14%, -2%) |
| 17 | 520 nM | (68%, 23%) |
| 18 | 10.8 nM | 5.8 µM |
| 19 | 10.5 nM | 11.7 µM |
| 20 | 7.56 nM | (96%, 45%) |
| 21 | 21.7 nM | (57%, 19%) |
| 22 | 33.7 nM | (75%, 28%) |
| 23 | 221 nM | (89%, 52%) |
| 24 | 48.6 nM | (80%, 36%) |
| 25 | 171 nM | (90%, 58%) |
| Nicotine | 1.6 nM | 182 µM |

| | | |
|---|---|---|
| ^{*1}: Values indicated in a parenthesis show control % of [³H]-cytisine biding at 1 µM and 10 µM of test compounds, respectively. | | |
| ^{**2}: Values indicated in a parenthesis show control % of [³H]-α-Bgt biding at 100 µM and 1,000 µM of test compounds. | | |

**TABLE 16:**

| Compound No. | Affinities for receptors Ki | |
|---|---|---|
| | α4β2 ^{*1} | α1β1γδ ^{**2} |
| 26 | 28.2 nM | 41.6 µM |
| 27 | 53.1 nM | 16.3 µM |
| 28 | 2.77 nM | 39.8 µM |
| 29 | 0.25 nM | 7.02 µM |
| 30 | 26.7 nM | 22.5 µM |
| 31 | 93 nM | (37%, 10%) |
| 32 | 10 nM | 14.6 µM |
| 33 | 32 nM | (15%, 1%) |
| 34 | 4.9 nM | (14%, -1%) |
| 35 | 41 nM | (12%, -3%) |
| 36 | 263 nM | (10%, 2%) |
| 37 | 16.4 nM | 22.9 µM |
| 38 | 10.6 nM | 65.2 µM |
| 39 | 30.5 nM | 10.8 µM |
| 40 | 355 nM | (71%, 35%) |
| 41 | 32 nM | (79%, 30%) |
| 42 | 290 nM | (75%, 35%) |
| 43 | 37.1 nM | 19.9 µM |
| 44 | 64 nM | (80%, 26%) |
| 45 | 143 nM | (18%, 6%) |
| 46 | 273 nM | (88%, 66%) |
| 47 | 227 nM | (93%, 73%) |
| 48 | 47.9 nM | 56.3 µM |
| 49 | (62%, 16%) | (18%, 14%) |
| 50 | 27.1 nM | 818 µM |
| Nicotine | 1.6 nM | 182 µM |

| | | |
|---|---|---|
| ^{*1}: Values indicated in a parenthesis show control % of [³H]-cytisine binding at 1 µM and 10 µM of test compounds, respectively. | | |
| ^{**2}: Values indicated in a parenthesis show control % of [³H]-α-Bgt binding at 100 µM and 1,000 µM of test compounds. | | |

**TABLE 17:**

| Compound No. | Affinities for receptors Ki | |
|---|---|---|
| | α4β2 ^{*1} | α1β1γδ ^{**2} |
| 51 | (96%, 33%) | (103%, 53%) |
| 52 | 24.9 nM | 302 µM |
| 53 | 226 nM | (98%, 56%) |
| 54 | 9.72 nM | (113%, 52%) |
| 55 | 43 nM | 66 µM |
| 56 | 165 nM | 545 µM |
| 57 | 11.9 nM | 13 µM |
| 58 | (62%, 16%) | (62%, 37%) |
| 59 | 50.2 nM | 1234 µM |
| 60 | 31.9 nM | 61.3 µM |
| 61 | 65.4 nM | 219 µM |
| 62 | 29.1 nM | 79.8 µM |
| 63 | 160 nM | 364 µM |
| 64 | (60%, 15%) | (77%, 23%) |
| 65 | 181 nM | 311 µM |
| 66 | 16.1 nM | 184 µM |
| Nicotine | 1.6 nM | 182 µM |

| | | |
|---|---|---|
| ^{*1}: Values indicated in a parenthesis show control % of [³H]-cytisine binding at 1 µM and 10 µM of test compounds, respectively. | | |
| ^{**2}: Values indicated in a parenthesis show control % of [³H]-α-Bgt binding at 100 µM and 1,000 µM of test compounds. | | |

### Biological Experiment 3:

### Agonist activities at human α4β2 subtype of nicotinic acetylcholine receptors

Agonist activities of the compounds of the present invention at human α4β2 subtype of nicotinic acetylcholine receptors was evaluated by the following method, which was modified method described by Papke R. L., Thinschmidt J. S., Moulton B. A., Meyer E. M. & Poirier A., *Br. J*. *Pharmacol*., 120, 429-438 (1997).

### (1) Preparation of cRNA of human α4β2 subtype of nicotinic acetylcholine receptors

Cloning of human nicotinic acetylcholine receptor (hnACh-R) α4 cDNA and hnAC-R β2 cDNA were performed, in accordance with the conventional manners, by synthesizing each DNA primers corresponding to the sequences of hnACh-R α4 cDNA and hnACh-R β2 cDNA [Monteggia L. M. et al., *Gene,* 155, 189-193 (1995); and Anand R., & Lindstrom J., *Nucl*. *Acids Res.,* 18, 4272 (1990)], and obtained hnACh-R α4 cDNA and hnACh-R β2 cDNA by polymerase chain reaction (PCR), respectively. Obtained hnACh-R α4 cDNA and hnACh-R β2 cDNA were inserted to the cRNA expression vector (pSP64 polyA) having SP6 RNA promoter to construct hnACh-R α4/pSP64 polyA and hnACh-R β2/pSP64 polyA, respectively. After cutting from expression vector by restriction enzyme (EcoRI), transcription was performed by affecting SP6 RNA polymerase in the presence of cap analogues to obtain hnACh-R α4 cRNA and hnACh-R β2 cRNA, respectively.

### (2) Expression of human α4β2 subtype nicotinic acetylcholine receptors in Xenopus oocytes

Oocytes were purchased from Kitanihonseibutsukyohzai Co., Ltd., which were already enucleated from *Xenopus laevis*, and used in this experiment.

The oocytes were treated with collagenase (Sigma type I; 1 mg/ml) in calcium-free modified Birth's solution (88 mM of NaCl, 1 mM of KCl, 2.4 mM of NaHCO₃, 0.82 mM of MgSO₄, 15 mM of HEPES, pH 7.6) under gently stirring at room temperature for 90 minutes, and washed out the enzyme from the tissue. Then, oocytes were separated from ovarian follicle by tweezers, and isolated oocytes were placed in antibiotics containing modified Birth's solution (88 mM of NaCl, 1 mM of KCl, 2.4 mM of NaHCO₃, 0.82 mM of MgSO₄, 15 mM of HEPES, pH 7.6, and 0.1 v/v% of mixture solution containing of penicillin and streptomycin for incubation; Sigma Co.). Thus treated oocytes were injected with 50 nl of adjusted cRNAs (1.0 mg/ml), that is, each 50 ng of hnACh-R α4 cRNA and hnACh-R β2 cRNA per 1 oocyte by using automatic injector (NANOJECT; DRUMMOND SCIENTIFIC CO.), and further incubated for 4-14 days at 19°C. In oocytes, heterogeneous quintuple [(α4)₂(β2)₃] was composed by translation of injected *cRNAs*, and ion channel receptors were constructed on cell membrane.

### (3) Agonist activities at human α4β2 subtype of nicotinic acetylcholine receptors

Recordings of response at human α4β2 subtype of nicotinic acetylcholine receptors by means of membrane potential holding method were performed as follow. That is, oocytes were placed in recording chamber with a total volume of 50 µl and were perfused with Ringer's solution (115 mM of NaCl, 2.5 mM of KCl, 1.8 mM of CaCl₂, 10 mM of HEPES, pH 7.3) containing atropine (1 µM) under flow rate of 1 ml/min. The membrane electric potentials were held at -50 mV by mean of two electric membranes potential holding method (CEZ-1250; Nihon Kohden Co.). Test compounds were added to the perfusion solution, and recorded the peak strength of induced inward current. In order to normalize the response of test compounds, the response with acetylcholine (Ach) were recorded before and after application of the test compounds. Generally in the oocytes just after isolated, the response of intrinsic muscarinic acetylcholine receptors, which is inward electric current caused by activation of calcium dependence chloride ion channels with increase of the intracellular calcium concentration by stimulation of receptors, is observed. However, the complete disappearance of the response was confirmed when treated with collagenase or added 1 µM of atropine. Furthermore, the oocytes without injection of cRNAs showed no response by Ach after treatment with collagenase. Therefore, the responses observed in oocytes with injection of hnACh-R α4 cRNA and hnACh-R β2 cRNA, i.e., the inward current induced by the intracellular influx of sodium ion according to the stimulation of receptors, would be the freshly observed responses of human α4β2 subtype nicotinic acetylcholine receptors.

Table 18 shows the results of agonist activity test of the compounds in the present invention and (-)-nicotine as reference compound.

**TABLE 18:**

| Compound No. | Agonist activity (ED50)^{*1} | Compound No. | Agonist activity (ED50)^{*1} |
|---|---|---|---|
| 1 | (20%) | 29 | 0.5 µM |
| 5 | (4.9%) | 31 | (4%) |
| 6 | 86.0 µM | 33 | (6%) |
| 7 | (16%) | 34 | (13%) |
| 8 | 4.2 µM | 44 | (10%) |
| 9 | 92.0 µM | 50 | 92.4 µM |
| 11 | (47%) | 55 | (17%) |
| 12 | (21%) | 56 | (11%) |
| 13 | 14.7 µM | 57 | (23%) |
| 14 | 27.1 µM | 59 | (21%) |
| 16 | 1.5 µM | 62 | 325 µM |
| 19 | (3%) | nicotine | 11.4 µM |
| 28 | 15.5 µM | | |

| | | | |
|---|---|---|---|
| ^{*1} : These date are shown in control % by response at 100 µM of the test compounds, in comparison with the response at 10 µM of acetylcholine (100%). Values indicated in a parenthesis show control % by response at 100 µM of the test compounds. | | | |

Following are Formulation Examples of the compounds (I) or pharmaceutically acceptable salt thereof according to the present invention

**Formulation Example 1 (Tablets):**

| | |
|---|---|
| Compound 16 | 25 g |
| Lactose | 130 g |
| Crystalline cellulose | 20 g |
| Corn starch | 20 g |
| 3% aqueous solution of hydroxypropylmethyl-cellulose | 100 ml |
| Magnesium stearate | 2 g |

Compound 16, lactose, crystalline cellulose and corn starch were screened through a 60-mesh sieve, homogenized and charged into a kneader. 3% aqueous solution of hydroxypropylmethylcellulose was added to the homogeneous mixture and the mixture was further kneaded. The product was granulated by a 16-mesh sieve, dried in air at 50°C, and again granulated by a 16-mesh sieve. Magnesium stearate was added to the granule and mixed again. The mixture was tabletted to produce tablets weighing 200 mg each and having an 8 mm diameter.

**Formulation Example 2 (Capsules):**

| | |
|---|---|
| Compound 28 | 25.0 g |
| Lactose | 125.0 g |
| Corn starch | 48.5 g |
| Magnesium stearate | 1.5 g |

Above components were finely pulverized and thoroughly mixed to produce a homogeneous mixture. The mixture was filled in gelatin capsules, 200 mg per capsule, to obtain capsules.

### Formulation Example 3 (Injection):

Hydrochloride of Compound 29 was filled in an amount of 250 mg in a vial and mixed in situ with approximately 4-5 ml of injectable distilled water to make an injectable solution.

### INDUSTRIAL APPLICABILITY

As described above, the compounds of the present invention possess high affinity to α4β2 nicotinic acetylcholine receptor of central nervous system and activate said α4β2 nicotinic acetylcholine receptors as agonists or modulators. Therefore, the compounds of the present invention are useful for preventing or treating various kinds of diseases, which may be prevented or cured by activating nicotinic acetylcholine receptors.

Especially, activators for α4β2 nicotinic acetylcholine receptors of the present invention are useful for preventing or treating various diseases such as dementia, senile dementia, presenile dementia, Alzheimer's disease, Parkinson's disease, cerebrovascular dementia, AIDS-related dementia, dementia in Down's syndrome, Tourette's syndrome, neurosis during chronic cerebral infarction stage, cerebral dysfunction caused by cerebral injury, anxiety, schizophrenia, depression, Huntington's disease, pain and so on.

## Claims

1. Activators for α4β2 nicotinic acetylcholine receptors containing heterocyclic compounds represented by the following formula (I): 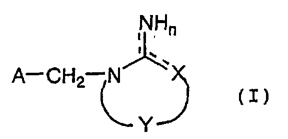 wherein:
A is optionally substituted aryl group; or optionally substituted heterocyclic group;
X is oxygen atom; sulfur atom; carbon atom; or nitrogen atom;
dotted line shows either presence or absence of bond; n is integer of 1 or 2; and Y is,
(1) in the case of X is oxygen atom, group -Y-X- is -CH₂-CH₂-O- or -CH₂-CH₂-CH₂-O-;
(2) in the case of X is sulfur atom, group -Y-X- is-CH(R¹)-CH₂-S-, -C(R²)=C(R³)-S- or -CH₂-CH₂-CH₂-S- (in which, R¹, R² and R³ are hydrogen atom; C₁-C₄ alkyl group; or optionally substituted phenyl group);
(3) in the case of X is carbon atom, group -Y-X- is -CH₂-CH₂-CH₂-, -CH=C(R⁴)-C(R⁵)=C(R⁶)-, -CH₂-CH₂-CH₂-CH₂-, or -N=C(R⁷)-CH=CH- (in which, R⁴, R⁵, R⁶ and R⁷ are hydrogen atom; C₁-C₄ alkyl group; optionally substituted phenyl group; halogen atom; or nitro group); and,
(4) in the case of X is nitrogen atom, group -Y-X- is-CH₂-CH₂-NH-, -CH₂-CH₂-CH₂-NH-, -CH=C(R⁸)-N= or -CH=C(R⁹)-CH=N- (in which, R⁸ and R⁹ are hydrogen atom; or optionally substituted phenyl group);
or pharmaceutically acceptable salts thereof as active ingredient.

2. The activators for α4β2 nicotinic acetylcholine receptors according to claim 1, wherein said activators are agonists or modulators at α4β2 nicotinic acetylcholine receptors.

3. A therapeutic agent for preventing or treating cerebral circulation diseases comprising the activator for α4β2 nicotinic acetylcholine receptors claimed in claim 1 or 2.

4. A therapeutic agent for preventing or treating neurodegenerative disease, dementia, motor ataxia, and neuropathy and mental disease comprising the activator for α4β2 nicotinic acetylcholine receptors claimed in claim 1 or 2.

5. The therapeutic agent according to claim 4, wherein said neurodegenerative disease is Alzheimer's disease or Parkinson's disease, said dementia is cerebrovascular dementia, said motor ataxia is Tourette's syndrome, and said neuropathy and mental disease is neurosis during chronic cerebral infarction stage, anxiety or schizophrenia.

6. A medicament for improving the cerebral metabolism, neurotransmission functional disorder and memory disorder, for protecting brain, or having analgesic effect, which comprises the activator for α4β2 nicotinic acetylcholine receptors claimed in claim 1 or 2.

7. A medicament for preventing or treating inflammatory intestinal diseases comprising the activator for α4β2 nicotinic acetylcholine receptors claimed in claim 1 or 2.

8. The use of the compounds claimed in claim 1 or pharmaceutically acceptable salts thereof as the activators for α4β2 nicotinic acetylcholine receptors.

9. The following compounds represented by the formula (I) of claim 1 or pharmaceutically acceptable salts thereof;
1-(6-chloro-3-pyridyl)methyl-2-iminoimidazolidine;
1-(6-chloro-3-pyridyl)methyl-2-iminopyrrolidine;
1-(6-chloro-3-pyridyl)methyl-2-iminopiperidine;
3-(6-chloro-3-pyridyl)methyl-2-imino-3,4,5,6-tetrahydro-2H-1,3-oxazine;
3-(6-chloro-3-pyridyl)methyl-2-imino-3,4,5,6-tetrahydro-2H-1,3-thiazine;
3-(6-fluoro-3-pyridyl)methyl-2-imino-4-methyl-2,3-dihydrothiazole;
3-(6-bromo-3-pyridyl)methyl-2-imino-4-methyl-2,3-dihydrothiazole;
3-(6-chloro-3-pyridyl)methyl-2-imino-4,5-dimethyl-2,3-dihydrothiazole;
3-(6-chloro-3-pyridyl)methyl-4-ethyl-2-imino-2,3-dihydrothiazole;
5-chloro-1-(6-chloro-3-pyridyl)methyl-2-imino-1,2-dihydropyridine;
1-(6-chloro-3-pyridyl)methyl-2-imino-3-methyl-1,2-dihydropyridine;
1-(6-chloro-3-pyridyl)methyl-2-imino-5-methyl-1,2-dihydropyridine;
1-(6-chloro-3-pyridyl)methyl-2-imino-4-methyl-1,2-dihydropyridine;
2-imino-1-(3-pyridyl)methyl-1,2-dihydropyridine;
3-(6-chloro-3-pyridyl)methyl-2-imino-4-methylthiazolidine;
3-(6-chloro-3-pyridyl)methyl-2-iminooxazolidine;
1-(6-chloro-3-pyridyl)methyl-2-imino-1,2,3,4,5,6-hexahydropyrimidine;
3-(5-bromo-3-pyridyl)methyl-2-imino-4-methyl-2,3-dihydrothiazole;
3-(4-chlorobenzyl)-2-iminothiazolidine;
2-imino-3-(6-methyl-3-pyridyl)methylthiazolidine;
2-imino-3-(4-pyridazinyl)methylthiazolidine;
3-(2-chloro-5-thiazolyl)methyl-2-iminothiazolidine;
2-imino-3-(3-methyl-5-isoxazolyl)methylthiazolidine;
2-imino-4-methyl-3-(3-methyl-5-isoxazolyl)methyl-2,3-dihydrothiazole;
3-(2-chloro-5-thiazolyl)methyl-2-imino-4-methyl-2,3-dihydrothiazole;
3-(5,6-dichloro-3-pyridyl)methyl-2-imino-4-methyl-2,3-dihydrothiazole;
2-imino-4-methyl-3-(6-methyl-3-pyridyl)methyl-2,3-dihydrothiazole;
3-(6-chloro-3-pyridyl)methyl-2-imino-5-phenyl-2,3-dihydrothiazole;
3-(6-chloro-3-pyridyl)methyl-2-imino-4-phenyl-2,3-dihydrothiazole;
4-(4-chlorophenyl)-3-(6-chloro-3-pyridyl)methyl-2-imino-2,3-dihydrothiazole;
3-(6-chloro-3-pyridyl)methyl-2-imino-4-phenylthiazolidine;
2-(6-chloro-3-pyridyl)methyl-3-imino-6-phenyl-2,3-dihydropyridazine;
3-imino-6-phenyl-2-(3-pyridyl)methyl-2,3-dihydropyridazine;
1-(6-chloro-3-pyridyl)methyl-2-imino-5-phenyl-1,2-dihydropyrimidine;
1-(6-chloro-3-pyridyl)methyl-2-imino-5-nitro-1,2-dihydropyridine;
2-imino-1-(6-methyl-3-pyridyl)methyl-1,2-dihydropyridine;
2-imino-3-(3-pyridazinyl)methylthiazolidine;
2-amino-1-(2-chloro-5-thiazolyl)methylimidazole;
2-amino-1-(6-chloro-3-pyridyl)methyl-4,5-dimethylimidazole;
2-amino-1-(5-pyrimidyl)methylimidazole;
2-amino-1-(6-chloro-3-pyridyl)methyl-4-methylimidazole;
2-amino-1-(5,6-dichloro-3-pyridyl)methylimidazole;
2-amino-1-(3-pyridyl)methylimidazole;
2-amino-1-(6-methyl-3-pyridyl)methylimidazole;
3-(4-chlorobenzyl)-2-imino-2,3-dihydrothiazole;
2-amino-1-(4-chlorobenzyl)imidazole;
2-amino-1-(7-aza-3-indolyl)methylimidazole;
3-(3,4-dichlorobenzyl)-2-imino-2,3-dihydrothiazole;
2-imino-3-(3-nitrobenzyl)-2,3-dihydrothiazole;
2-imino-3-(4-nitrobenzyl)-2,3-dihydrothiazole;
2-imino-3-(4-methylbenzyl)-2,3-dihydrothiazole;
2-imino-3-(3-trifluoromethylbenzyl)-2,3-dihydrothiazole;
3-(4-cyanobenzyl)-2-imino-2,3-dihydrothiazole;
3-(7-aza-3-indolyl)-2-imino-2,3-dihydrothiazole;

10. Activators for α4β2 nicotinic acetylcholine receptors containing compound claimed in claim 9 or pharmaceutically acceptable salts thereof as active ingredient.

11. The activators for α4β2 nicotinic acetylcholine receptors according to claim 10, wherein said activators are agonists or modulators at α4β2 nicotinic acetylcholine receptors.

12. A therapeutic agent for preventing or treating cerebral circulation diseases comprising the activator for α4β2 nicotinic acetylcholine receptors claimed in claim 10 or 11.

13. A therapeutic agent for preventing or treating neurodegenerative disease, dementia, motor ataxia, and neuropathy and mental disease comprising the activator for α4β2 nicotinic acetylcholine receptors claimed in claim 10 or 11.

14. The therapeutic agent according to claim 13, wherein said neurodegenerative disease is Alzheimer's disease or Parkinson's disease, said dementia is cerebrovascular dementia, said motor ataxia is Tourette's syndrome, and said neuropathy and mental disease is neurosis during chronic cerebral infarction stage, anxiety or schizophrenia.

15. A medicament for improving the cerebral metabolism, neurotransmission functional disorder and memory disorder, for protecting brain, or having analgesic effect, which comprises the activator for α4β2 nicotinic acetylcholine receptors claimed in claim 10 or 11.

16. A medicament for preventing or treating inflammatory intestinal diseases comprising the activator for α4β2 nicotinic acetylcholine receptors claimed in claim 10 or 11.

17. The use of the compounds claimed in claim 9 or pharmaceutically acceptable salts thereof as the activators for α4β2 nicotinic acetylcholine receptors.
